# EUROPEAN PATENT APPLICATION

(11) **EP 4 084 107 A1**
(43) Date of publication of application: **02.11.2022**
(21) Application number: 20907185.1
(22) Date of filing: 10.12.2020
(51) Int. Cl.: H01L 51/50, C09K 11/06

(54) **ORGANIC ELECTROLUMINESCENT ELEMENT AND METHOD FOR MANUFACTURING SAME**

(30) Priority: 27.12.2019 JP 2019238763
(71) Applicant: NIPPON STEEL Chemical & Material Co., Ltd., Tokyo 103-0027 (JP)
(72) Inventor: HAYASHI, Kentaro, Tokyo 103-0027 (JP); OGAWA, Junya, Tokyo 103-0027 (JP); IKENAGA, Yuji, Tokyo 103-0027 (JP)
(74) Representative: Vossius & Partner Patentanwälte Rechtsanwälte mbB
(86) International application number: PCT/JP2020/046099
(87) International publication number: WO 2021/131755

(57) **Abstract**

To provide an organic electroluminescent device realizing high efficiency and low voltage characteristics. A light-emitting layer including compounds of (1) and (2) and a dopant is included. X represents N or CR³, wherein at least one X represents N. R¹ represents (1b), R² represents (1c), and R³ represents an alkyl group or the like. Y represents O, S, NR⁴, or CR⁵R⁶, R⁴ to R⁶ each represent an alkyl group or the like, and one of R⁷ to R¹⁴ binds to a ring in (1). * represents a binding position to a ring in (1), and rings C and D are each an aromatic ring fused to an adjacent ring. R¹⁵, R¹⁶ and R¹⁷ have the same meaning as R³ (a and b represent the number of substitutions) . R¹⁹ has the same meaning as R⁴, and R²⁰ to R²⁷ each represent CR³, CR²⁸ or N and at least one thereof represents CR²⁸, and R²⁸ represents formula (2b). Z represents O, S, NR³⁷, or CR³⁸R³⁹, R³⁷ to R³⁹ each has the same meaning as R⁴, and one of R²⁹ to R³⁶ binds to a ring in (2) and the others represent CR³ or N.

## Description

### Technical Field

The present invention relates to an organic electroluminescent element or device (hereinafter referred to as an organic EL device), more specifically relates to an organic EL device comprising a specific mixed host material.

### Background Art

Application of a voltage to an organic EL device allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

Further, highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency and low voltage characteristics are required.

In addition, patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%.

However, all the mechanisms have room for advancement in terms of both efficiency and life, and are additionally required to be improved also in terms of reduction in driving voltage.

Meanwhile, Patent Literatures 3 and 4 disclose use of an indolocarbazole compound with a substituted fused heterocycle, as a host material.

Patent Literatures 5 and 6 disclose use of an indolocarbazole compound as a mixed host.

However, none of these can be said to be sufficient, and further improvements in efficiency and voltage are desired.

### Citation List

### Patent Literature

Patent Literature 1: WO2010/134350
Patent Literature 2: WO2011/070963
Patent Literature 3: WO2013/137001
Patent Literature 4: WO2013/122402
Patent Literature 5: WO2016/023608
Patent Literature 6: WO2018/198844

### Summary of Invention

### Technical Problem

In order to apply organic EL devices to display devices such as flat panel displays, it is necessary to improve the luminous efficiency of devices and at the same time, to sufficiently secure the stability during driving. In view of the above circumstances, an object of the present invention is to provide a practically useful organic EL device realizing a high efficiency and low voltage characteristics.

As a result of intensive studies, the present inventors have found that the above problem can be solved by an organic EL device using a specific mixed host material in a light-emitting layer, and have completed the present invention.

The present invention is an organic EL device having a plurality of organic layers between an anode and a cathode opposed to each other, wherein the organic layers comprise at least one light-emitting layer, and the light-emitting layer comprises a compound represented by the general formula (1), a compound represented by the general formula (2) and a dopant.

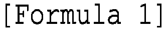

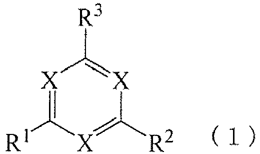

In the formula, X represents N or CR³, wherein at least one X represents N. R¹ is represented by the following formula (1b), and R² is represented by the following formula (1c). R³ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other. Herein, when the group has a hydrogen atom, the hydrogen atom is optionally replaced with deuterium or halogen.

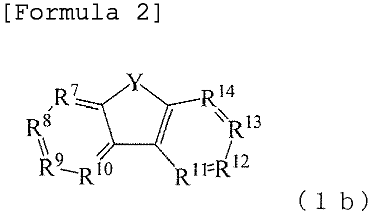

In the formula, Y represents O, S, NR⁴, or CR⁵R⁶. R⁴ to R⁶ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other. Herein, when the group has a hydrogen atom, the hydrogen atom is optionally replaced with deuterium or halogen. Any one of R⁷ to R¹⁴ represents a carbon atom binding to a ring of the general formula (1) and the others each independently represent CR³ or N, R³ is as described above and R³, when present in a plurality, may be the same or different.

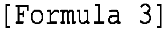

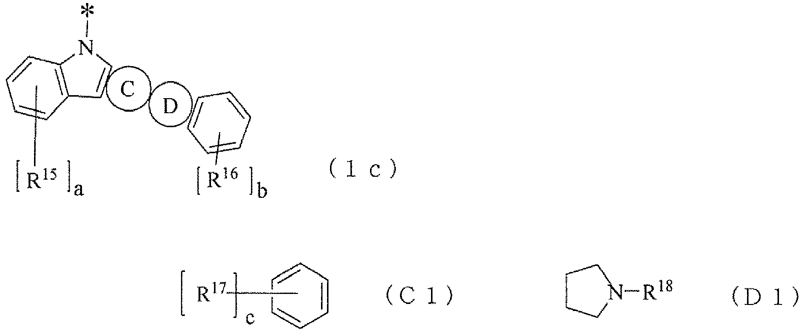

In the formula, * represents a position for binding to a ring of the general formula (1) and a ring C represents an aromatic ring fused at any positions of two adjacent rings and represented by formula (C1). A ring D represents a 5-membered ring fused at any positions of two adjacent rings and represented by formula (D1). R¹⁵, R¹⁶ and R¹⁷ each independently have the same meaning as R³, and when each present in a plurality, may be each the same or different. a, b and c represent the number of substitutions, a and b each independently represent an integer of 1 to 4, and c represents an integer of 1 to 2. R¹⁸ has the same meaning as R⁴.

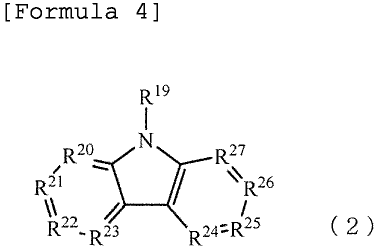

In the formula, R¹⁹ has the same meaning as R⁴. R²⁰ to R²⁷ each independently represent CR³, CR²8 or N, R³ is as described above, wherein at least one thereof is represented by CR²⁸, and R²⁸ is represented by the following formula (2b). R³ and R²⁸, when each present in a plurality, may be each the same or different.

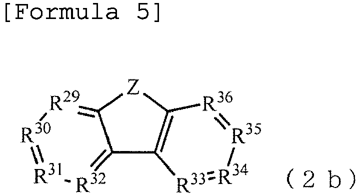

In the formula, Z represents O, S, NR³⁷, or CR³⁸R³⁹, and R³⁷ to R³⁹ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other. Herein, when the group has a hydrogen atom, the hydrogen atom is optionally replaced with deuterium or halogen. Any one of R²⁹ to R³⁶ represents a carbon atom binding to a ring of the general formula (2) and the others each independently represent CR³ or N. R³ is as described above and R³, when present in a plurality, may be the same or different.

Any of R⁷ or R⁸ in the formula (1b) is preferably linked to a ring of the general formula (1). Further preferably R⁸ is preferably linked to a ring of the general formula (1).

Y in the formula (1b) preferably represents O or S.

The formula (1c) is preferably represented by any of the following formulas (11) to (15).

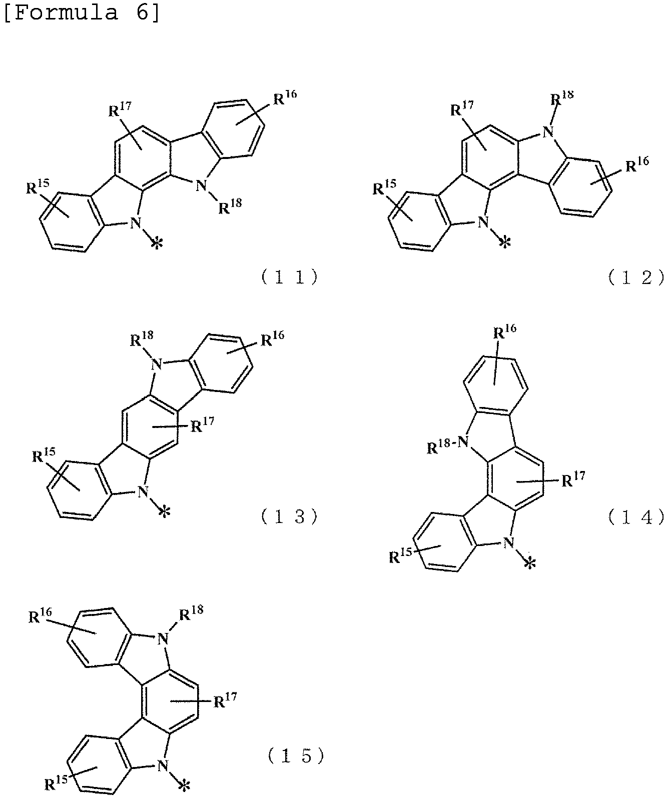

In the formulas, R¹⁵ to R¹⁸ have the same meaning as in the formula (1c).

The general formula (2) is preferably represented by any of the following general formulas (21) to (29): 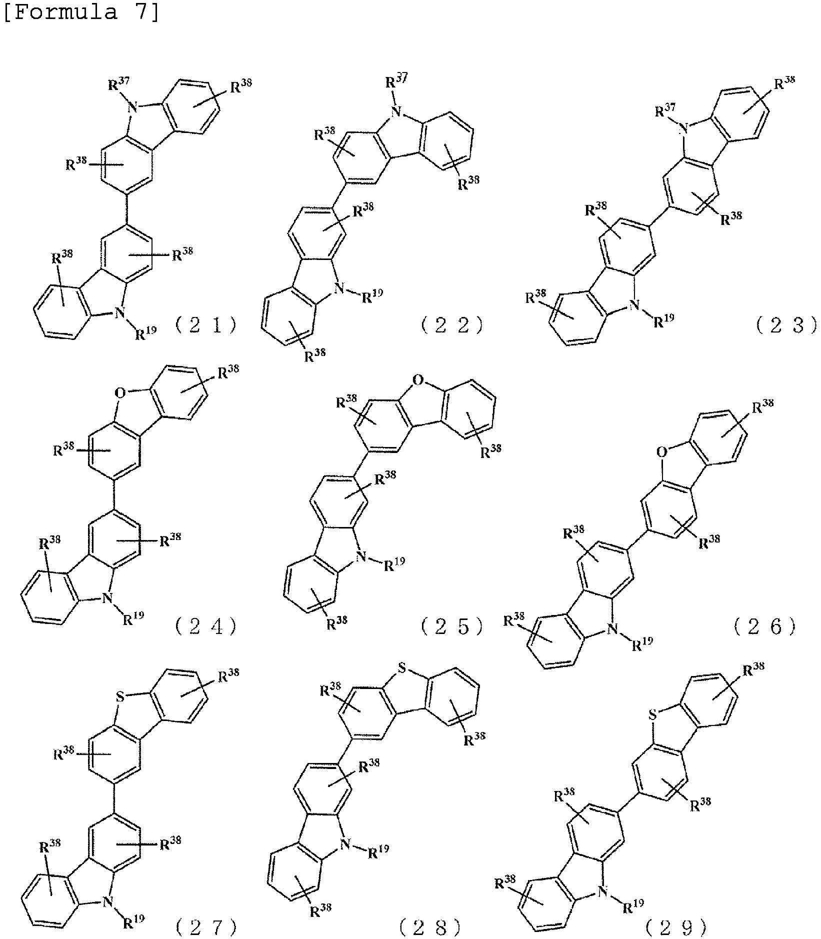 wherein R¹⁹ has the same meaning as in the general formula (2), and R³⁷ and R³⁸ have the same meaning as in the formula (2b).

The general formula (2) is more preferably represented by any of the general formulas (21) to (23).

In the general formulas (21) to (23), R¹⁹, R³⁷ and R³⁸ each preferably represent a substituted or unsubstituted aromatic hydrocarbon group having 6 to 10 carbon atoms, or a substituted or unsubstituted linked aromatic group in which two to five of these aromatic rings are linked to each other.

Hereinafter, preferred aspects of the organic EL device of the present invention are shown. In other words, the organic EL device according to the present invention has a mixed host including two compounds, and also includes a light-emitting layer having a dopant. The proportion of the compound represented by the general formula (1) based on the compound represented by the general formula (1) and the compound represented by the general formula (2) in total in the mixed host is preferably 10 wt% or more and less than 70 wt%, more preferably 20 wt% or more and less than 60 wt%. The light-emitting dopant is more preferably an organic metal complex containing at least one metal selected from the group consisting of ruthenium, rhodium, palladium, silver, rhenium, osmium, iridium, platinum and gold, or a thermally activated delayed fluorescence-emitting dopant.

In addition, production of the organic EL device preferably has a step of mixing the compound represented by the general formula (1) and the compound represented by the general formula (2) to prepare a premixture, and vapor-depositing a host material containing the premixture to form a light-emitting layer.

In the above method for producing the organic EL device, it is suitable that a difference in 50% weight reduction temperatures of the first host and the second host is within 20°C.

### Advantageous Effects of Invention

For improvement of device characteristics, a higher durability of a material used for an organic layer against charges is needed, and particularly, in the light-emitting layer, it is important to reduce leakage of excitons and charges to surrounding layers. For leakage reduction of charges/excitons, it is effective to improve localization of a light emission area in the light-emitting layer. For this purpose, it is necessary to control amounts of both charges (electrons/holes) injected into the light-emitting layer or amounts of both charges transported in the light-emitting layer within a preferred range.

The compound represented by the general formula (1) used in the present invention has a structure in which two or more different fused aromatic groups are bonded to a nitrogen-containing 6-membered ring. Thus, an organic EL device can be produced which is enhanced in charge transport properties, and durability against charges, and which is stably driven even at a low voltage. Meanwhile, a carbazole compound represented by the general formula (2) especially has high hole injection transport ability, and changing the binding form of a carbazole ring or the kind or number of substituents to a skeleton thereof allows a high-level control of hole injection transport properties. Then, a combination with the compound represented by the general formula (1) can control amounts of both charges injected into an organic layer within a preferred range, so that localization of a light emission area in the light-emitting layer can be improved. In particular, in the case of a delayed fluorescence-emitting EL device or a phosphorescent EL device, they have a lowest excited triplet energy sufficiently high to confine the excitation energy generated in the light-emitting layer and therefore, an organic EL device having no outflow of energy from the inside of the light-emitting layer and having high efficiency and extended life at a low voltage can be produced.

### Brief Description of Drawing

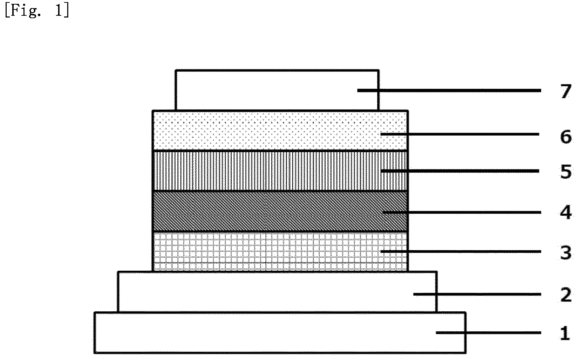

[Figure 1] Figure 1 is a schematic cross-sectional view showing one example of an organic EL device.

### Description of Embodiments

An organic EL device of the present invention has a structure wherein an anode, an organic layer and a cathode are laminated on a substrate, wherein at least one layer of the organic layer includes a light-emitting layer formed by using a predetermined material for an organic EL device. In other words, this organic EL device has an organic layer composed of a plurality of layers between the anode and the cathode opposed to each other; and at least one layer of the plurality of layers is a light-emitting layer, and there may be a plurality of light-emitting layers. Then, at least one of the light-emitting layers is a light-emitting layer composed of a vapor deposition layer containing a compound represented by the general formula (1) (hereinafter, referred to as "first host"), a compound represented by the general formula (2) (hereinafter, referred to as "second host"), and a light-emitting dopant.

In other words, the first host contained in the light-emitting layer is selected from the compounds represented by the general formula (1), and the second host contained therein is selected from the compounds represented by the general formula (2).

In particular, in the general formula (1), X represents N or CR³, wherein at least one X represents N. At least one X represents N, two or more X preferably represent N, all of X more preferably represent N. R¹ is represented by formula (1b), and R² is represented by formula (1c).

In the general formula (1), R³ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other. Herein, when the group has a hydrogen atom, the hydrogen atom is optionally replaced with deuterium or halogen, and preferred is a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other and more preferred is a substituted or unsubstituted aromatic hydrocarbon group having 6 to 18 carbon atoms, a substituted or unsubstituted aromatic heterocyclic group having 3 to 12 carbon atoms, or a substituted or unsubstituted linked aromatic group in which two to five of these aromatic hydrocarbon groups and aromatic heterocyclic groups are linked to each other.

In the present specification, the linked aromatic group refers to a group in which aromatic rings of an aromatic hydrocarbon group and/or an aromatic heterocyclic group are linked by a single bond. Specifically, two to five of the aromatic rings of the substituted or unsubstituted aromatic hydrocarbon group having 6 to 24 carbon atoms are linked, two to five of the aromatic rings of the substituted or unsubstituted aromatic heterocyclic group having 3 to 17 carbon atoms are linked, or two to five of the aromatic rings of the aromatic hydrocarbon group and the aromatic rings of the aromatic heterocyclic group are linked. These may be linked linearly or linked in a branched manner, and such aromatic rings may be the same or different.

In the formula (1b), Y represents O, S, NR⁴, or CR⁵R⁶. Y preferably represents O, S, or NR⁴, more preferably represents O or S.

R⁴ to R⁶ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other. Herein, when the group has a hydrogen atom, the hydrogen atom is optionally replaced with deuterium or halogen, and preferred is a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other and more preferred is a substituted or unsubstituted aromatic hydrocarbon group having 6 to 18 carbon atoms, a substituted or unsubstituted aromatic heterocyclic group having 3 to 12 carbon atoms, or a substituted or unsubstituted linked aromatic group in which two to five of these aromatic rings are linked to each other.

Any one of R⁷ to R¹⁴ represents a carbon atom binding to a ring of the general formula (1) and the others each independently represent CR³ or N, and R³ is as described above and R³, when present in a plurality, may be the same or different. Two or less of R⁷ to R¹⁴ each preferably represent N, and zero or one thereof more preferably represents N.

In the formula (1b), any of R⁷ or R⁸ is preferably linked to a ring of the general formula (1), R⁸ is more preferably linked to a ring of the general formula (1).

In the formula (1c), * represents a position for binding to the general formula (1) and a ring C represents an aromatic ring fused at any positions of two adjacent rings and represented by formula (C1). A ring D represents a 5-membered ring fused at any positions of two adjacent rings and represented by formula (D1).

R¹⁵, R¹⁶ and R¹⁷ each independently have the same meaning as R³, and R¹⁵, R¹⁶ and R¹⁷ in the formula (1c), when each present in a plurality, may be each the same or different.

In the formula (D1), R¹⁸ has the same meaning as R⁴.

All a and b in the formula (1c) and c in the formula (C1) represent the number of substitutions, a and b each independently represent an integer of 1 to 4, and c represents an integer of 1 to 2. Preferably, a and b is an integer of 1 to 2, and c is 1.

The second host is represented by the general formula (2).

In the general formula (2), R¹⁹ has the same meaning as R³.

In the general formula (2), R²⁰ to R²⁷ each independently represent CR³, CR²⁸ or N, and R³ is as described above, wherein at least one thereof is represented by CR²⁸. Two or less of R²⁰ to R²⁷ each preferably represent N, and zero or one thereof more preferably represents N.

In particular, R²⁸ is represented by formula (2b). R and R²⁸, when each present in a plurality, may be each the same or different.

In the formula (2b), Z represents O, S, NR³⁷, or CR³⁸R³⁹, and R³⁷ to R³⁹ each independently have the same meaning as R⁴. Any one of R²⁹ to R³⁶ represents a carbon atom binding to a ring of the general formula (2) and the others each independently represent CR³ or N. Two or less of R²⁹ to R³⁶ each preferably represent N, and zero or one thereof more preferably represents N. R³ is as described above and R³, when present in a plurality, may be the same or different.

In formulas (21) to (23), R¹⁹ and R³⁷ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 18 carbon atoms, or a substituted or unsubstituted linked aromatic group in which two to five of these aromatic rings are linked to each other, and when the group has a hydrogen atom, the hydrogen atom is optionally replaced with deuterium or halogen, and preferred is a substituted or unsubstituted aromatic hydrocarbon group having 6 to 24 carbon atoms, a substituted or unsubstituted aromatic heterocyclic group having 3 to 18 carbon atoms, or a substituted or unsubstituted linked aromatic group in which two to five of these aromatic rings are linked to each other and more preferred is a substituted or unsubstituted aromatic hydrocarbon group having 6 to 10 carbon atoms, or a substituted or unsubstituted linked aromatic group in which two to five of these aromatic rings are linked to each other.

The formulas (21) to (29) preferably each have at least one p-BiPh (biphenyl) group, and any of R¹⁹, R³⁷ and R³⁸ in the formulas (21) to (29) more specifically has a p-BiPh group.

In the formulas (21) to (23), R³⁸ each independently represent hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 18 carbon atoms, or a substituted or unsubstituted linked aromatic group in which two to five of these aromatic rings are linked to each other, and preferred is hydrogen, deuterium, an aromatic hydrocarbon group having 6 to 24 carbon atoms, a substituted or unsubstituted aromatic heterocyclic group having 3 to 18 carbon atoms, or a substituted or unsubstituted linked aromatic group in which two to five of these aromatic rings are linked to each other and more preferred is hydrogen, deuterium, a substituted or unsubstituted aromatic hydrocarbon group having 6 to 10 carbon atoms, or a substituted or unsubstituted linked aromatic group in which two to five of these aromatic rings are linked to each other.

In the case of R¹⁹, R³⁷ and R³⁸ being an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, or an alkylsulfonyl group having 1 to 20 carbon atoms, specific examples thereof include alkyl groups such as methyl, ethyl, propyl, butyl, pentyl, cyclopentyl, hexyl, cyclohexyl, heptyl, octyl, nonyl, decyl, undecyl, dodecyl, tridecyl, tetradecyl, pentadecyl, hexadecyl, heptadecyl, octadecyl, nonadecyl, and icosyl, aralkyl groups such as phenylmethyl, phenylethyl, phenylicosyl, naphthylmethyl, anthranylmethyl, phenanthrenylmethyl, and pyrenylmethyl, alkenyl groups such as vinyl, propenyl, butenyl, pentenyl, decenyl, and icosenyl, alkynyl groups such as ethynyl, propargyl, butynyl, pentynyl, decynyl, and icosynyl, dialkylamino groups such as dimethylamino, ethylmethylamino, diethylamino, dipropylamino, dibutylamino, dipentynylamino, didecylamino, and diicosylamino, diarylamino groups such as diphenylamino, naphthylphenylamino, dinaphthylamino, dianthranylamino, diphenanthrenylamino, and dipyrenylamino, diaralkylamino groups such as diphenylmethylamino, diphenylethylamino, phenylmethylphenylethylamino, dinaphthylmethylamino, dianthranylmethylamino, and diphenanthrenylmethylamino, acyl groups such as acetyl, propionyl, butyryl, valeryl, and benzoyl, acyloxy groups such as acetyloxy, propionyloxy, butyryloxy, valeryloxy, and benzoyloxy, alkoxy groups such as methoxy, ethoxy, propoxy, butoxy, pentoxy, hexoxy, heptoxy, octoxy, nonyloxy, and decanyloxy, alkoxycarbonyl groups such as methoxycarbonyl, ethoxycarbonyl, propoxycarbonyl, butoxycarbonyl, and pentoxycarbonyl, alkoxycarbonyloxy groups such as methoxycarbonyloxy, ethoxycarbonyloxy, propoxycarbonyloxy, butoxycarbonyloxy, and pentoxycarbonyloxy, alkylsulfoxy groups such as methylsulfonyl, ethylsulfonyl, propylsulfonyl, butylsulfonyl, and pentylsulfonyl, and a cyano group, a nitro group, a fluoro group, and a tosyl group. Preferred is an alkyl group having 1 to 12 carbon atoms, an aralkyl group having 7 to 20 carbon atoms, an alkoxy group having 1 to 10 carbon atoms, a diarylamino group having two aromatic hydrocarbon groups each having 6 to 15 carbon atoms, a cyano group, a fluoro group, or a tosyl group.

In the case of R¹⁹, R³⁷ and R³⁸ being an aromatic hydrocarbon group or an aromatic heterocyclic group, specific examples thereof include an aromatic group generated by removing one H from benzene, naphthalene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, benzothiadiazole, dibenzofuran, dibenzothiophene, dibenzoselenophene, or carbazole. Preferred examples include an aromatic group generated from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, oxadiazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, or benzothiadiazole. More preferred examples include an aromatic group generated from benzene, pyridine, pyrimidine, triazine, thiophene, isothiazole, thiazole, pyridazine, pyrrole, pyrazole, imidazole, triazole, thiadiazole, pyrazine, furan, isoxazole, oxazole, or oxadiazole.

Specific examples of the compound represented by the general formula (1) are shown below, but are not limited thereto.

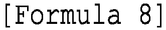

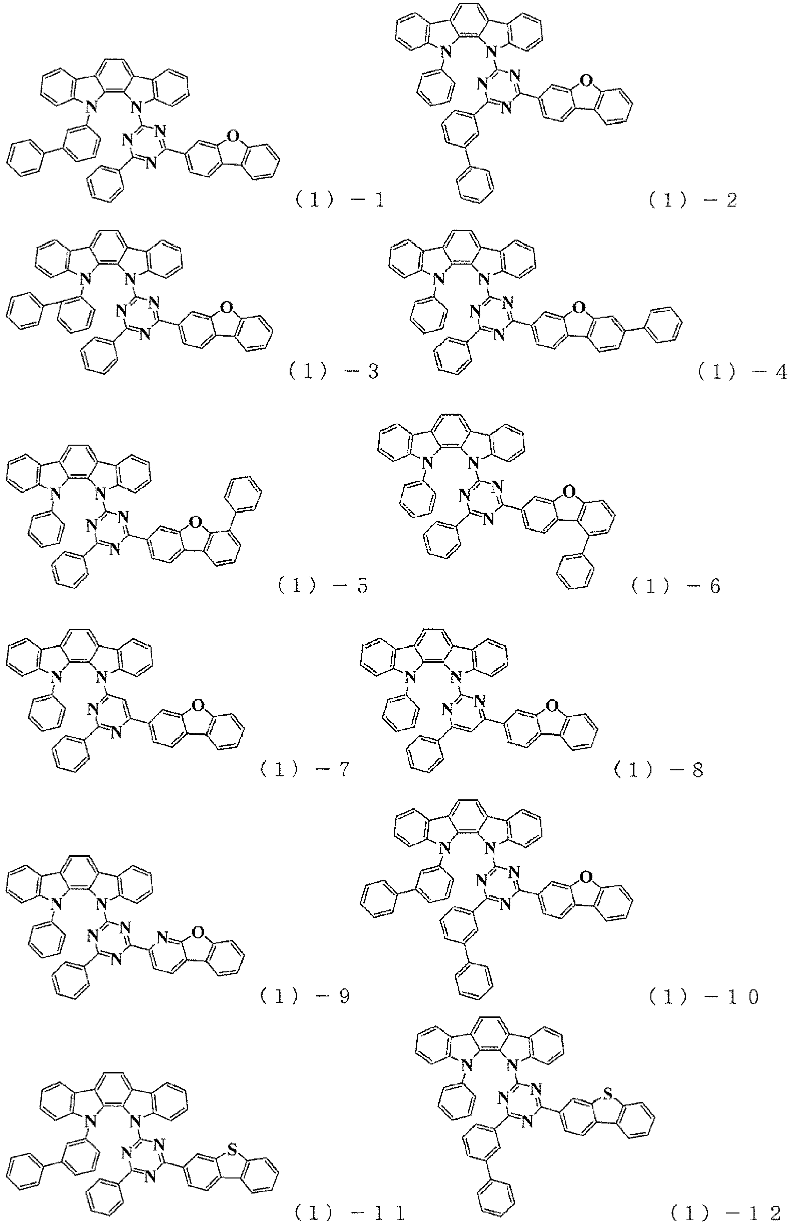

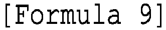

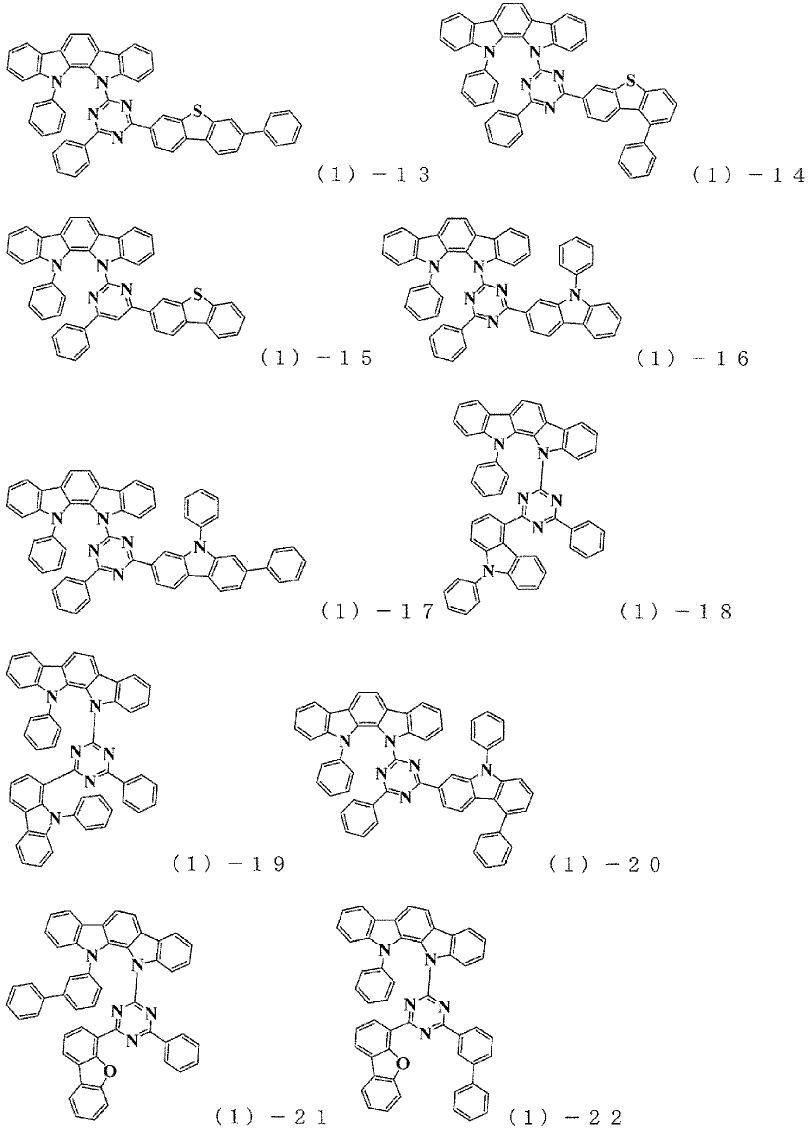

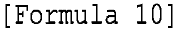

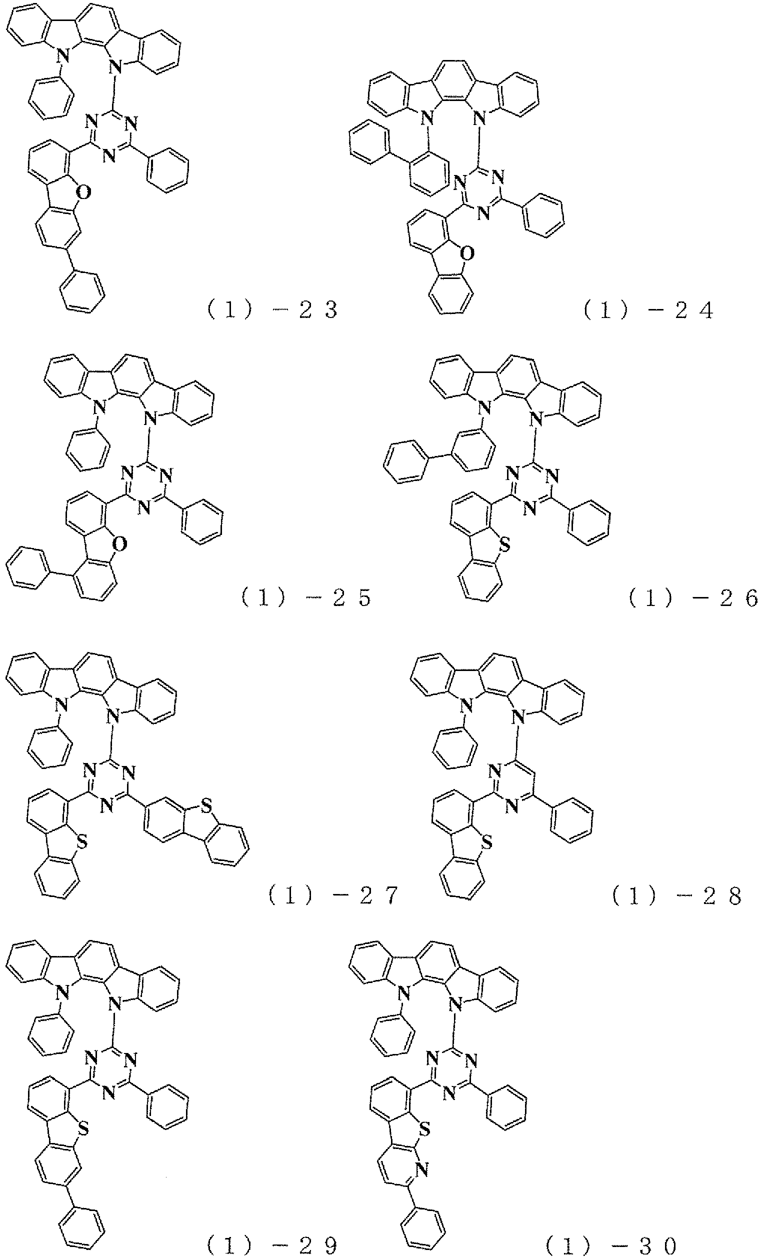

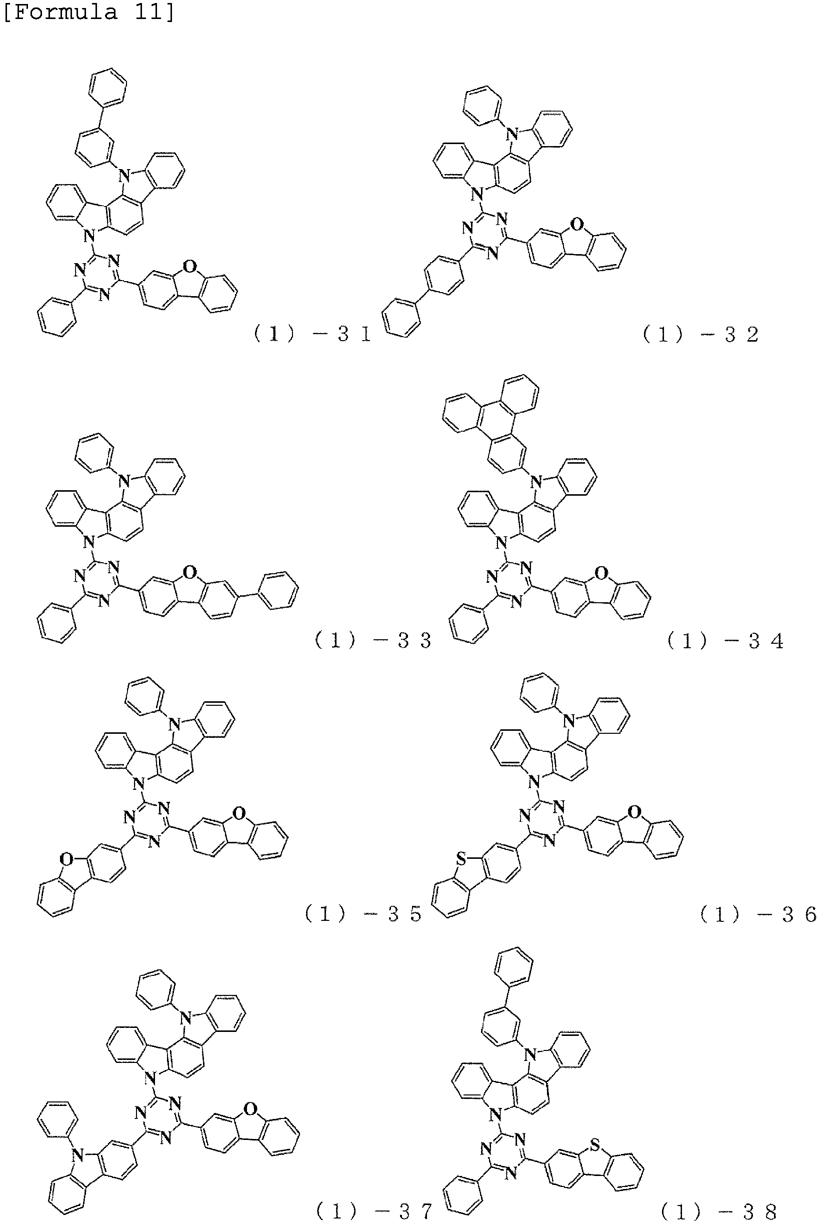

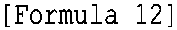

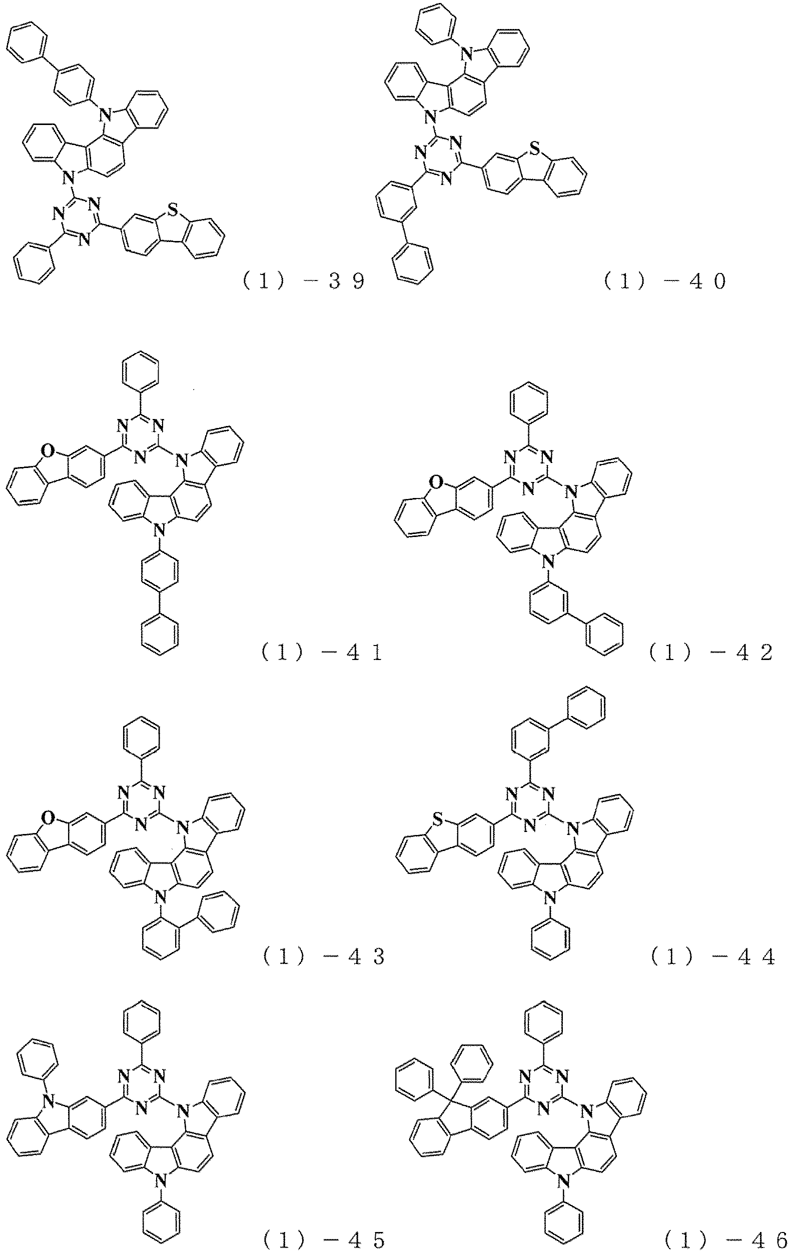

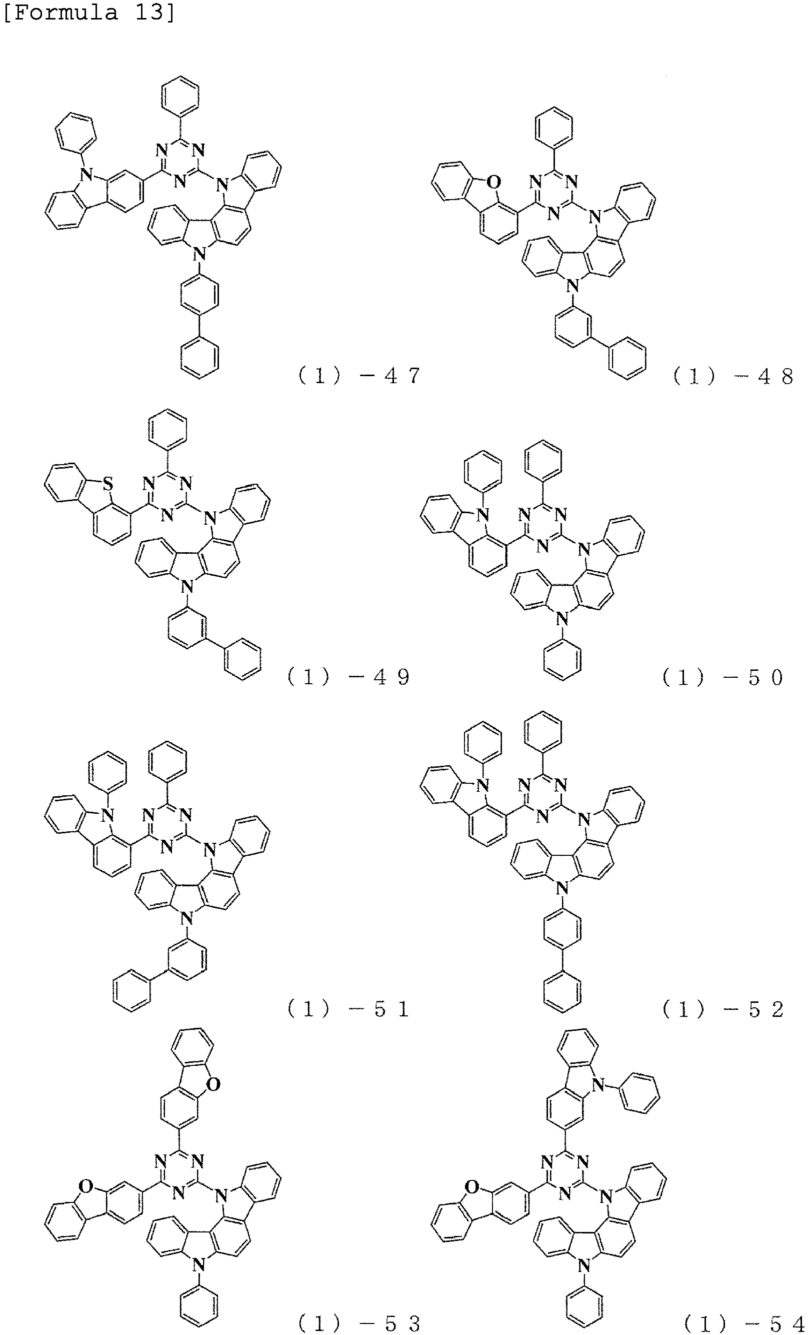

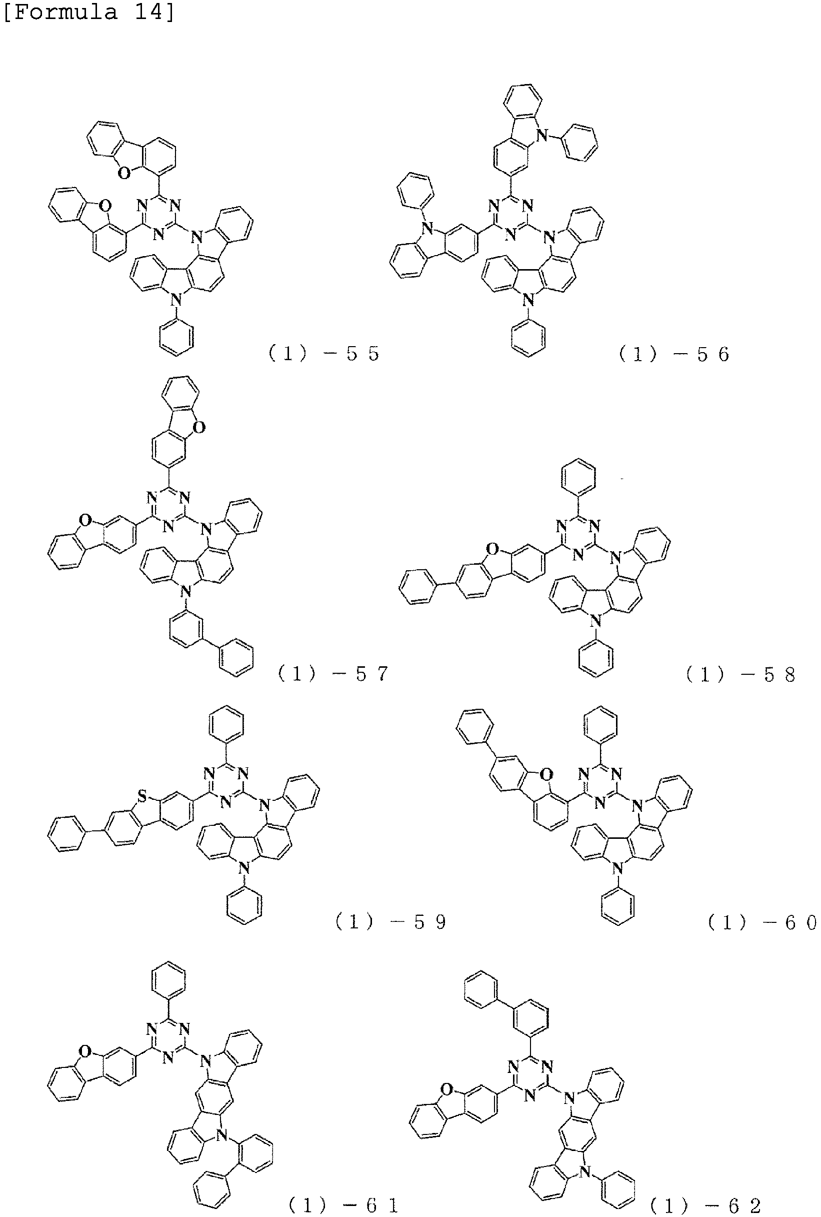

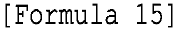

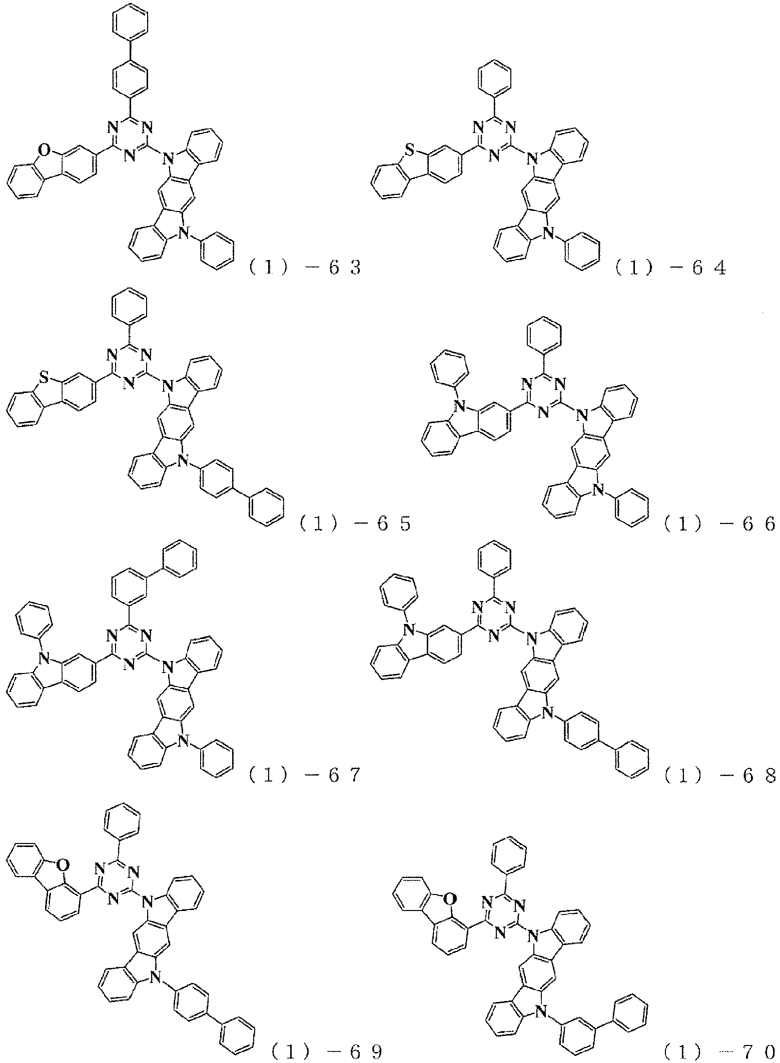

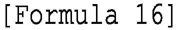

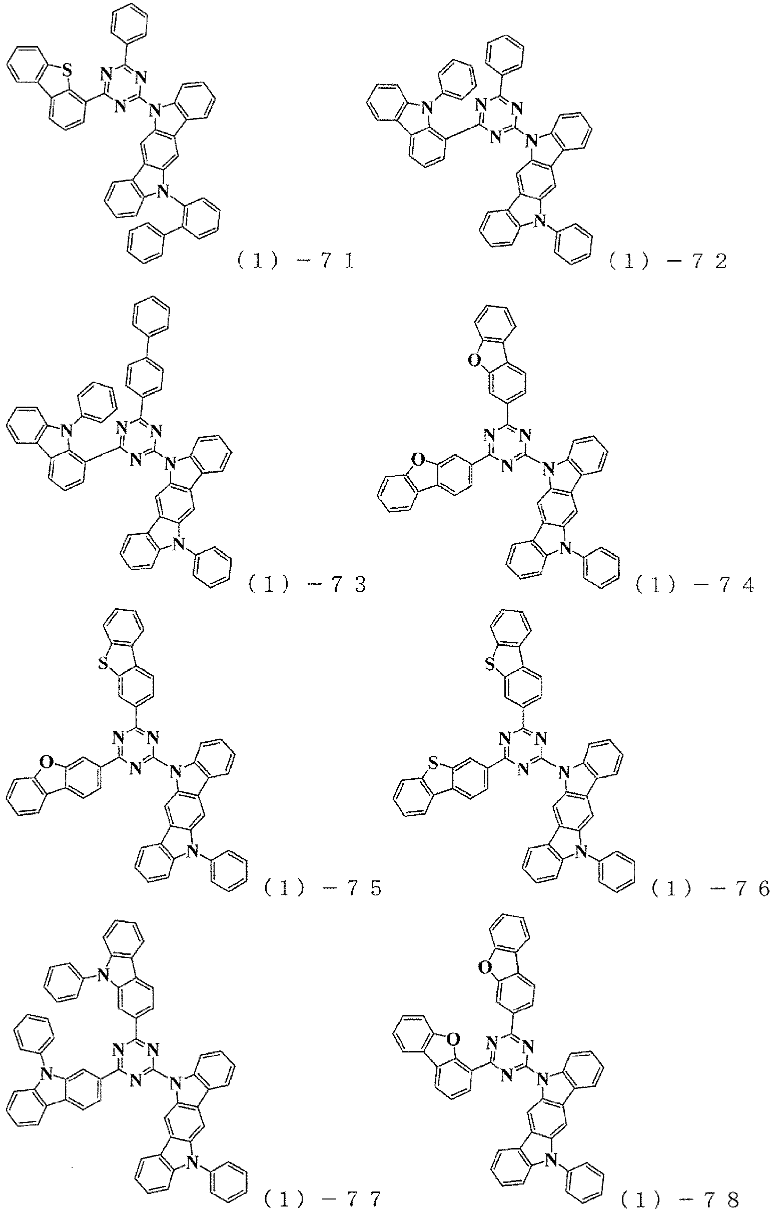

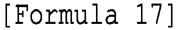

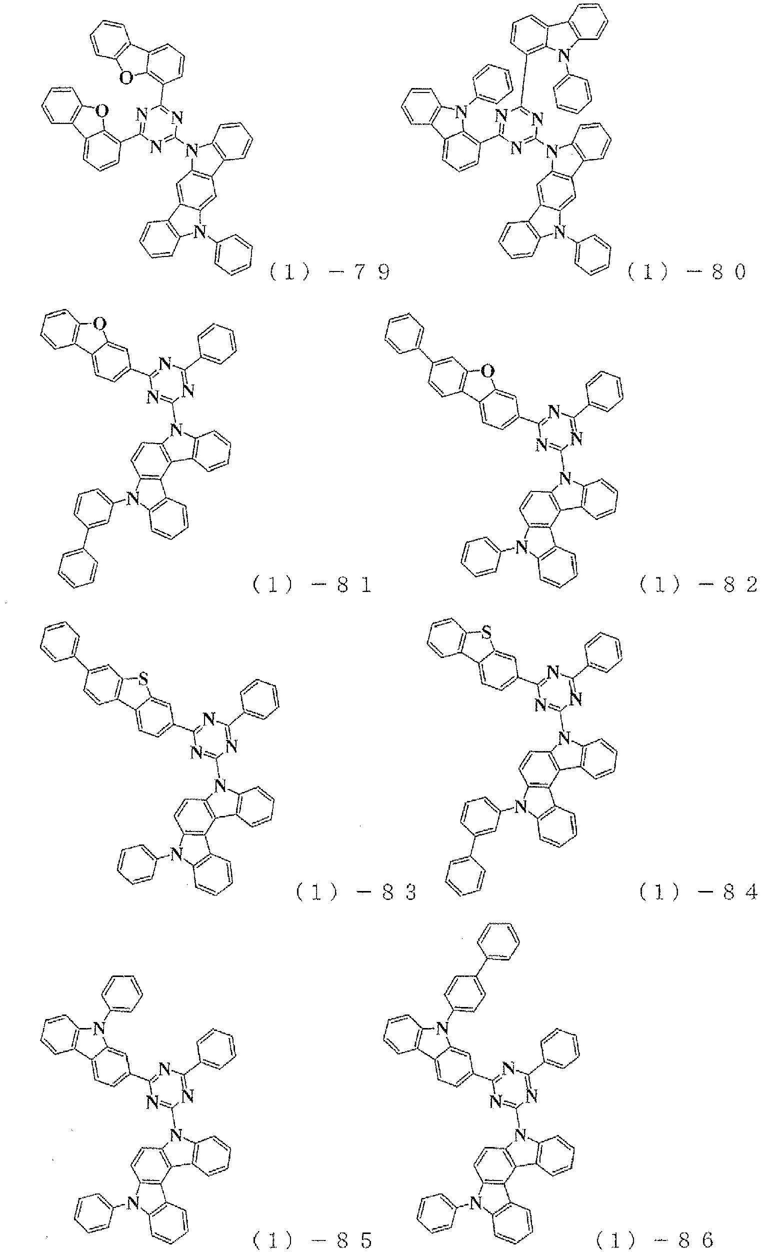

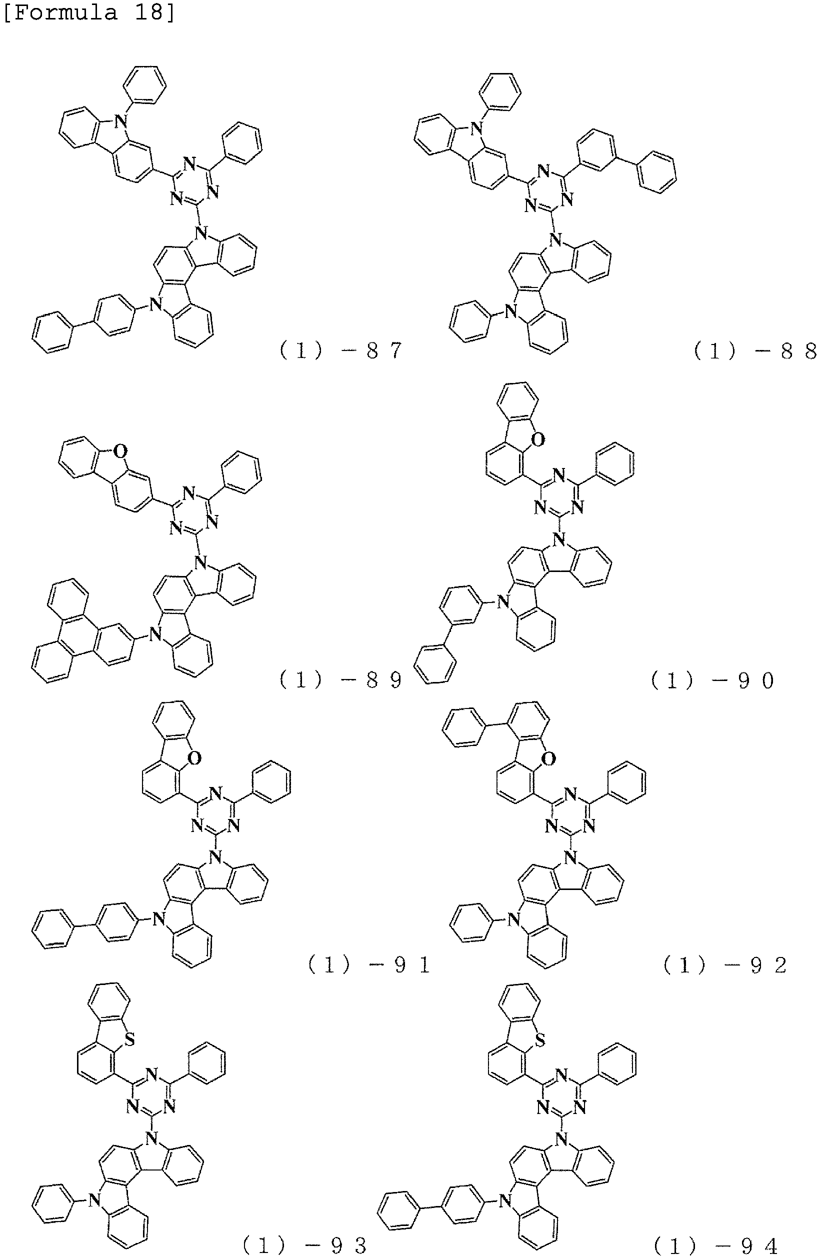

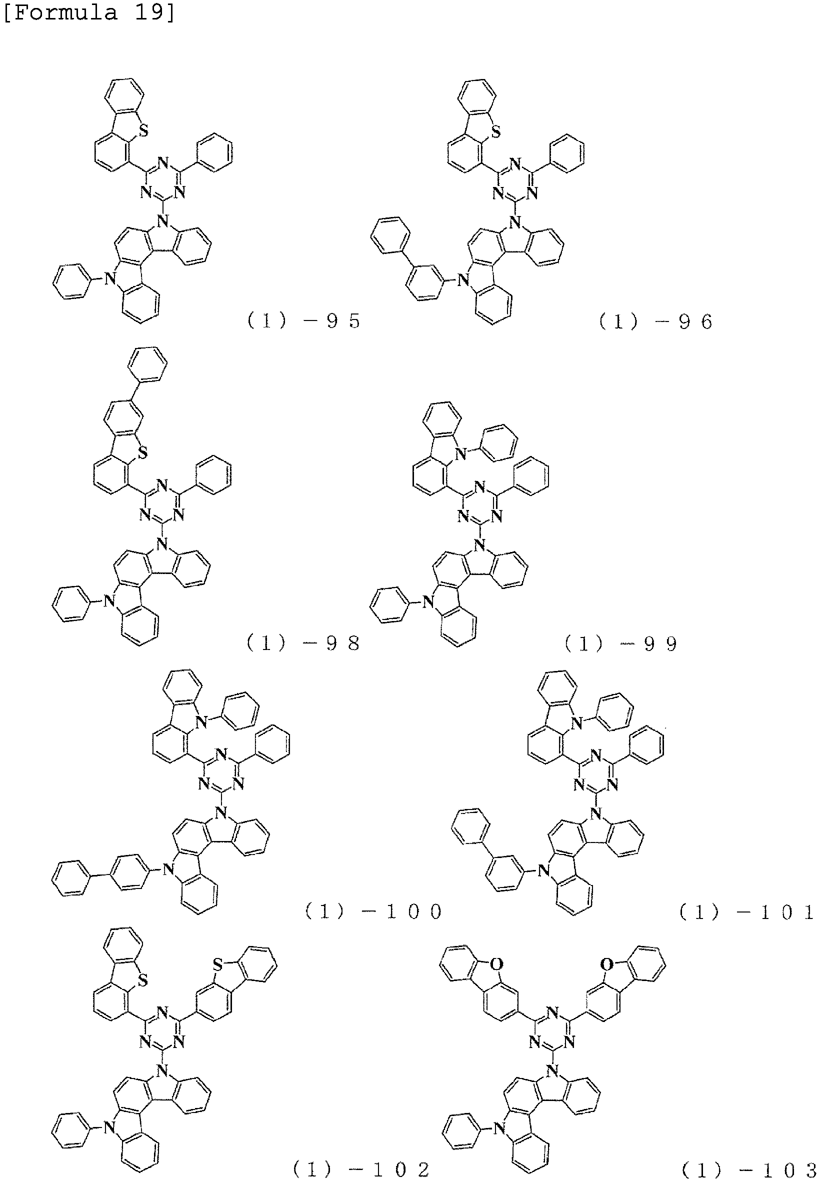

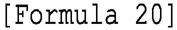

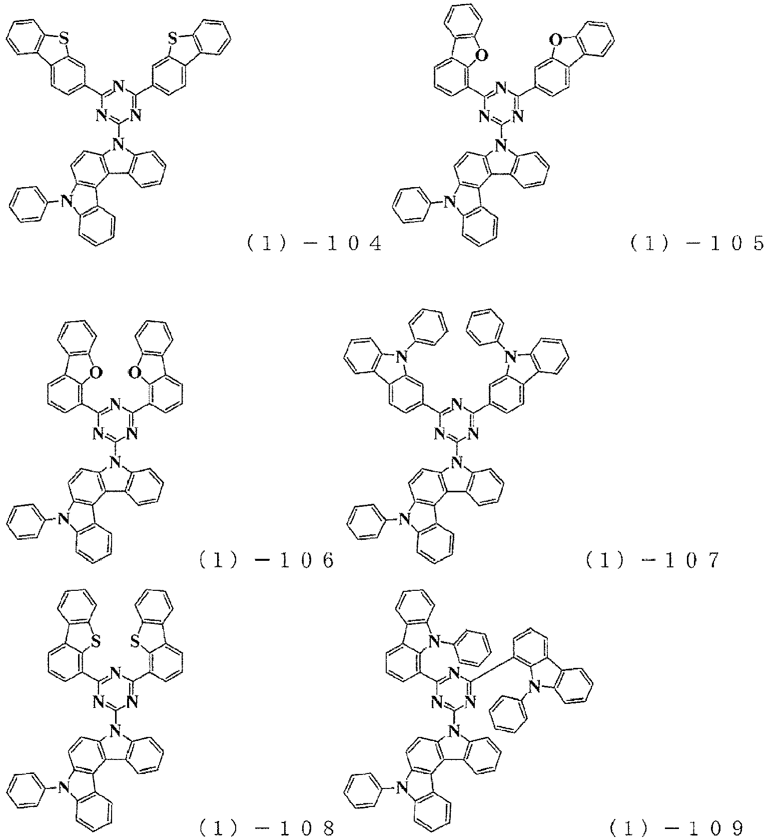

Specific examples of the compound represented by the general formula (2) are shown below, but are not limited thereto.

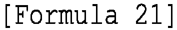

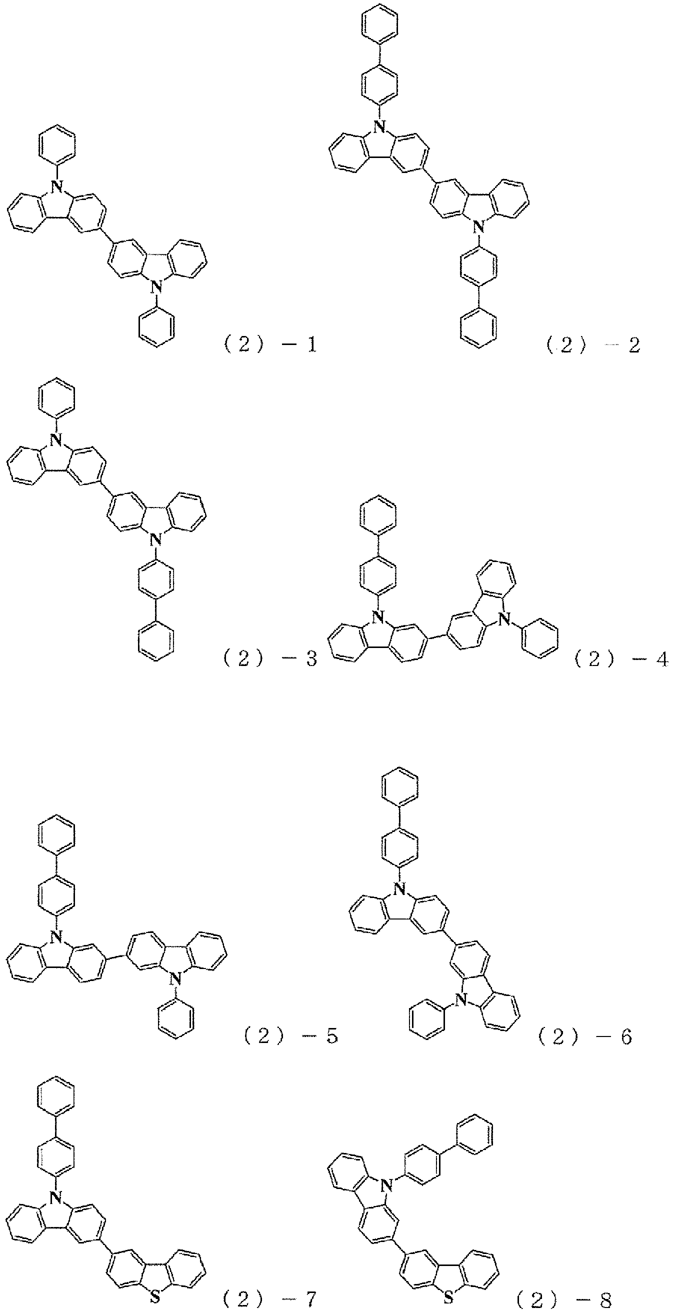

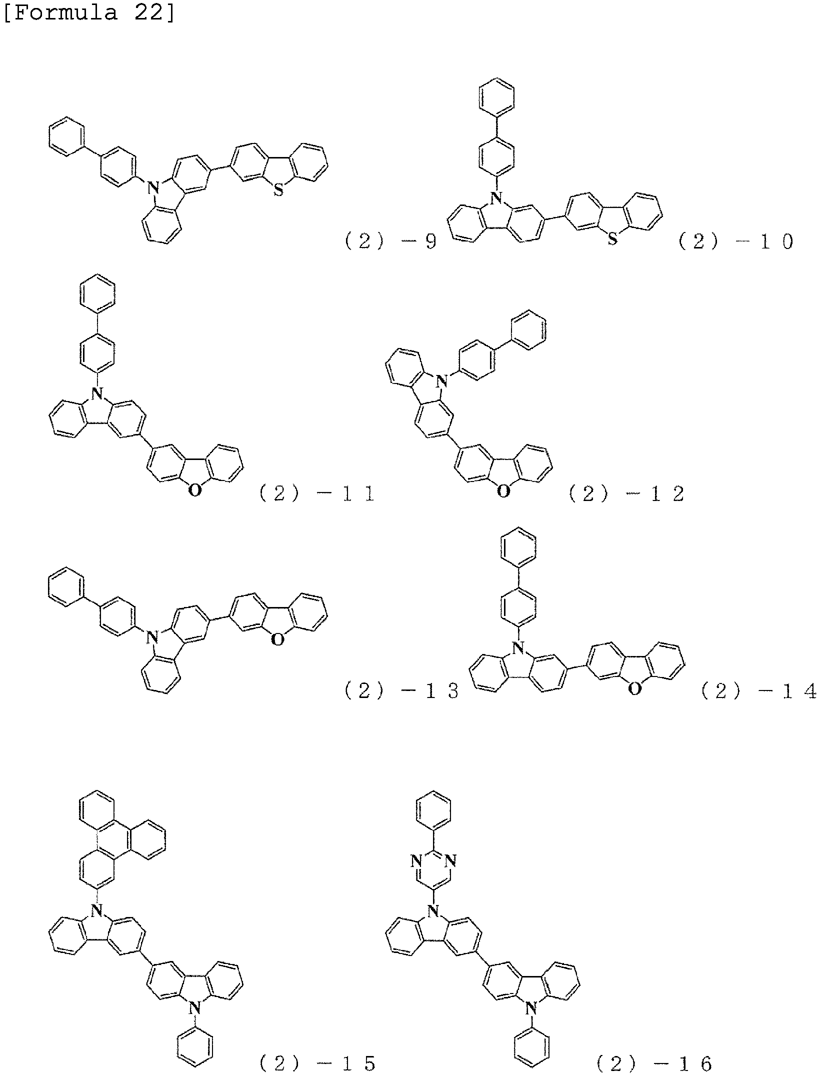

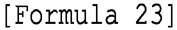

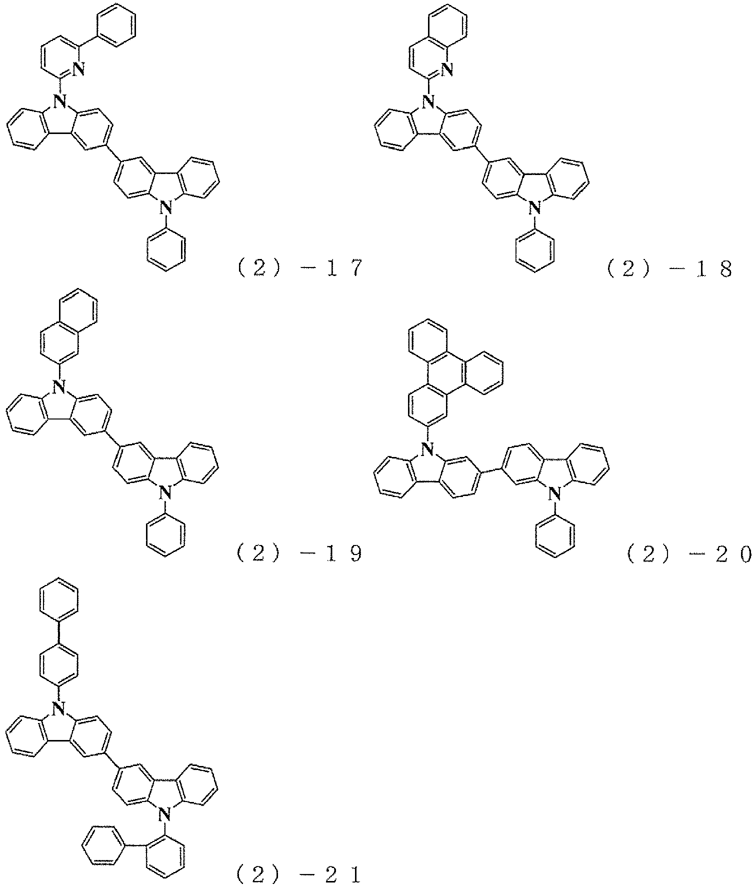

The light-emitting layer in the organic EL device of the present invention contains the compound represented by the general formula (1), the compound represented by the general formula (2), and the dopant, and may contain an aliphatic organic compound, an aromatic hydrocarbon compound, an aromatic heterocycle compound, an organic metal complex, and/or the like as other component(s). In the case of such other component(s) being contained, the proportion of such other compound(s) in total in the light-emitting layer is preferably less than 30 wt%, more preferably less than 10 wt%.

The proportion of the compound represented by the general formula (1) based on the compound represented by the general formula (1) and the compound represented by the general formula (2) in total is preferably 10 wt% or more and less than 70 wt%, more preferably 20 wt% or more and less than 60 wt%.

The light-emitting dopant is preferably an organic metal complex containing at least one metal selected from the group consisting of ruthenium, rhodium, palladium, silver, rhenium, osmium, iridium, platinum and gold, or a thermally activated delayed fluorescence-emitting dopant material.

The light-emitting layer in the organic EL device of the present invention may be formed by vapor deposition from a vapor deposition source, or may be formed by dissolution or dispersion in a solvent and a spin coating method, a bar coating method, a spraying method, an inkjet method, a printing method, or the like.

The light-emitting layer in the organic EL device of the present invention, if formed according to a vapor deposition method, can be formed by vapor deposition from different individual vapor deposition sources, but the light-emitting layer is preferably formed by obtaining a premixture by premixing before vapor deposition and vapor-depositing the premixture simultaneously from one vapor deposition source. In this case, the premixture may be mixed with a light-emitting dopant material necessary for formation of a light-emitting layer, or another host to be used as necessary. However, when there is a large difference in temperatures to provide desired vapor pressure, vapor deposition may be performed from another vapor deposition source.

The light-emitting layer in the organic EL device of the present invention, if formed by a printing method, can be formed according to the printing method by dissolving or dispersing the compound represented by the general formula (1), the compound represented by the general formula (2) and the dopant in a solvent to provide an ink for the light-emitting layer.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the anode side and the cathode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds **Ω**/□ or less. The film thickness is selected usually within 10 to 1000 nm, preferably within 10 to 200 nm though depending on the material.

### - Cathode -

Meanwhile, regarding a cathode material, preferable to a material of a metal (an electron injection metal), an alloy, an electrically conductive compound, or a mixture thereof, each having a small work function (4 eV or less) are used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds **Ω**/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm on the cathode, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. The light-emitting layer contains the compound represented by the general formula (1), the compound represented by the general formula (2), and a light-emitting dopant material.

The compound represented by the general formula (1) and the compound represented by the general formula (2) are each preferably used as a host material of the light-emitting layer. Regarding the compound represented by the general formula (1), one kind thereof may be used, or two or more kinds thereof may be used. Similarly, regarding the compound represented by the general formula (2), one kind thereof may be used, or two or more kinds thereof may be used.

If necessary, one, or two or more known host materials may be used in combination; however, it is preferable that an amount thereof to be used be 50 wt% or less, preferably 25 wt% or less based on the host materials in total of the compound represented by the general formula (1) and the compound represented by the general formula (2).

When the compound represented by the general formula (1) and the compound represented by the general formula (2) are premixed and used, it is desirable that a difference in 50% weight reduction temperature (T₅₀) between the compounds be small in order to produce an organic EL device having favorable characteristics with high reproducibility. The 50% weight reduction temperature is a temperature at which the weight is reduced by 50% when the temperature is raised to 550°C from room temperature at a rate of 10°C/min in TG-DTA measurement under a nitrogen stream reduced pressure (1 Pa). It is considered that vaporization due to evaporation or sublimation the most vigorously occurs around this temperature.

The difference in 50% weight reduction temperatures of the compound represented by the general formula (1) and the compound represented by the general formula (2) is preferably within 30°C, more preferably within 20°C. Regarding a premixing method, a known method such as pulverization and mixing can be used, and it is desirable to mix them as uniformly as possible. The difference in 50% weight reduction temperatures is herein expressed by an absolute value.

In the case of use of a plurality of kinds of hosts, such respective hosts can be vapor-deposited from different vapor deposition sources or can be simultaneously vapor-deposited from one vapor deposition source by premixing the hosts before vapor deposition to provide a premixture.

The premixing method is desirably a method that can allow for mixing as uniformly as possible, and examples thereof include pulverization and mixing, a heating and melting method under reduced pressure or under an atmosphere of an inert gas such as nitrogen, and sublimation, but not limited thereto.

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304 and Japanese Translation of PCT International Application Publication No. 2013-53051 are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.1 to 30 wt% and more preferably 1 to 20 wt% with respect to the host material.

The phosphorescent dopant material is not particularly limited, and specific examples thereof include the following.

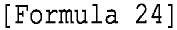

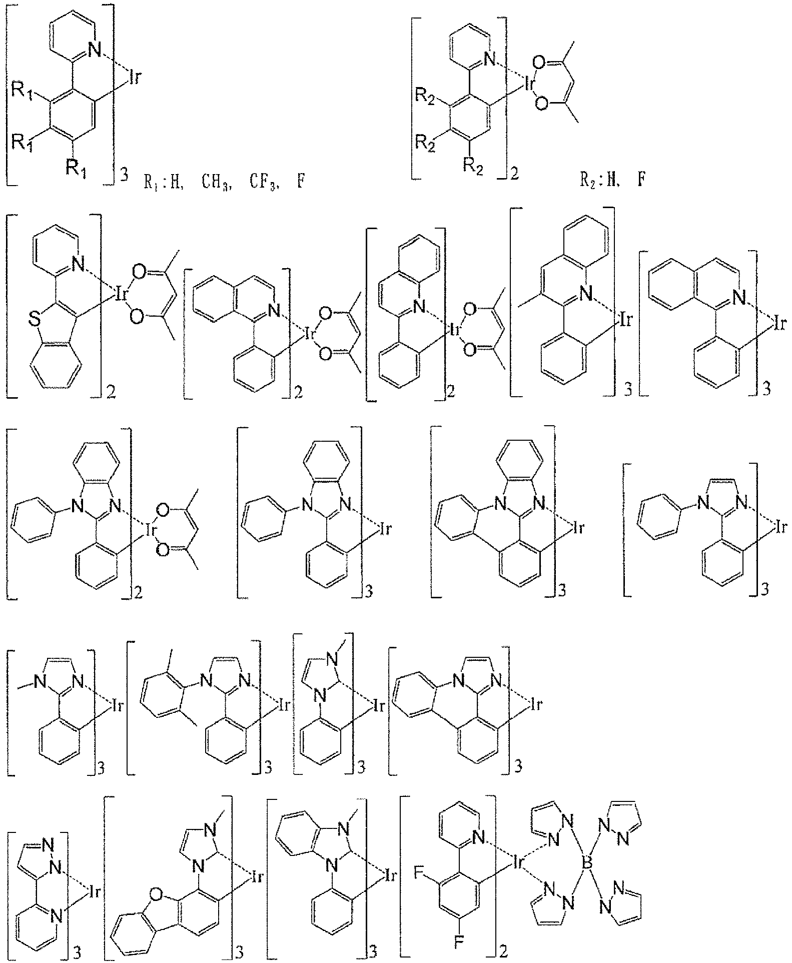

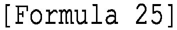

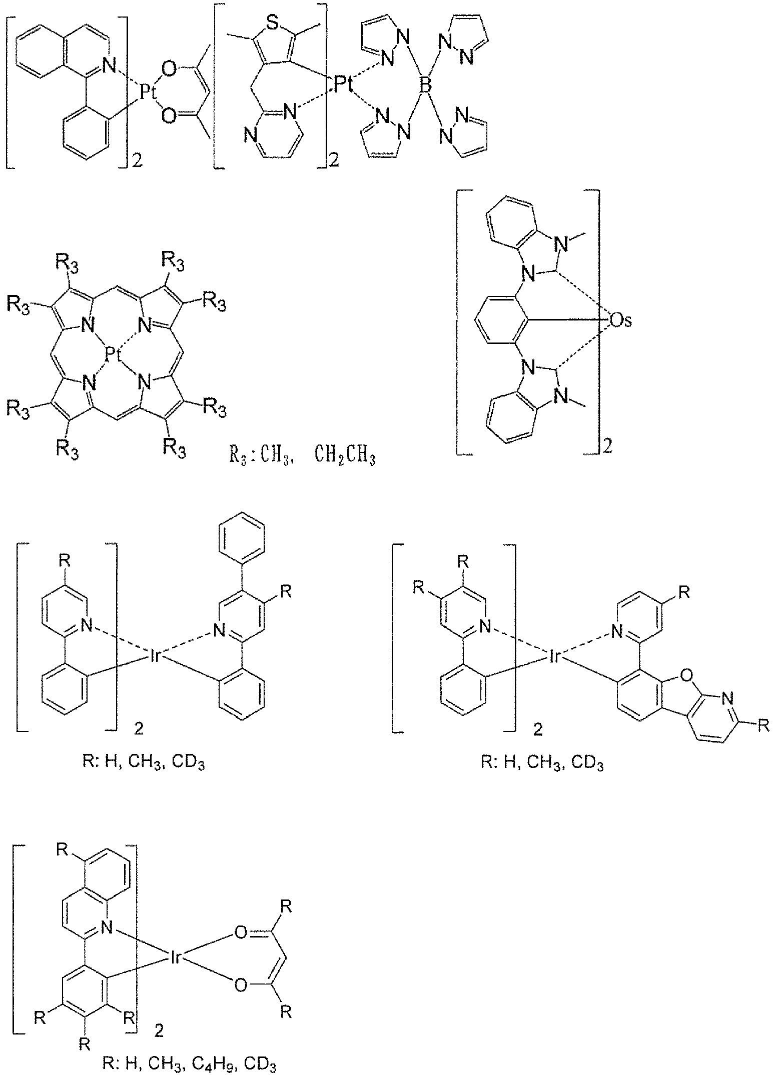

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, **α**-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.1% to 20% and more preferably 1% to 10% with respect to the host material.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

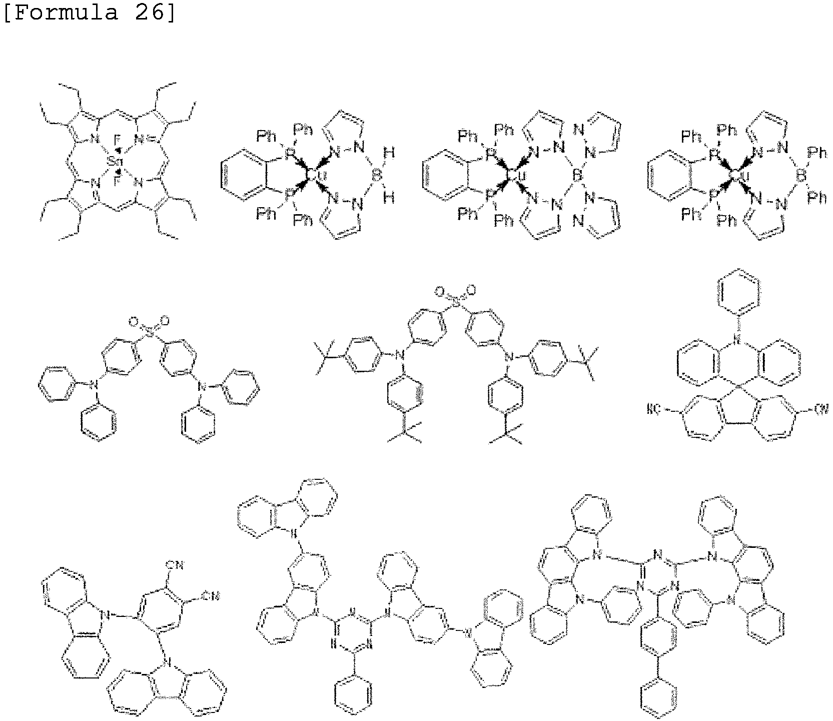

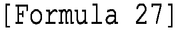

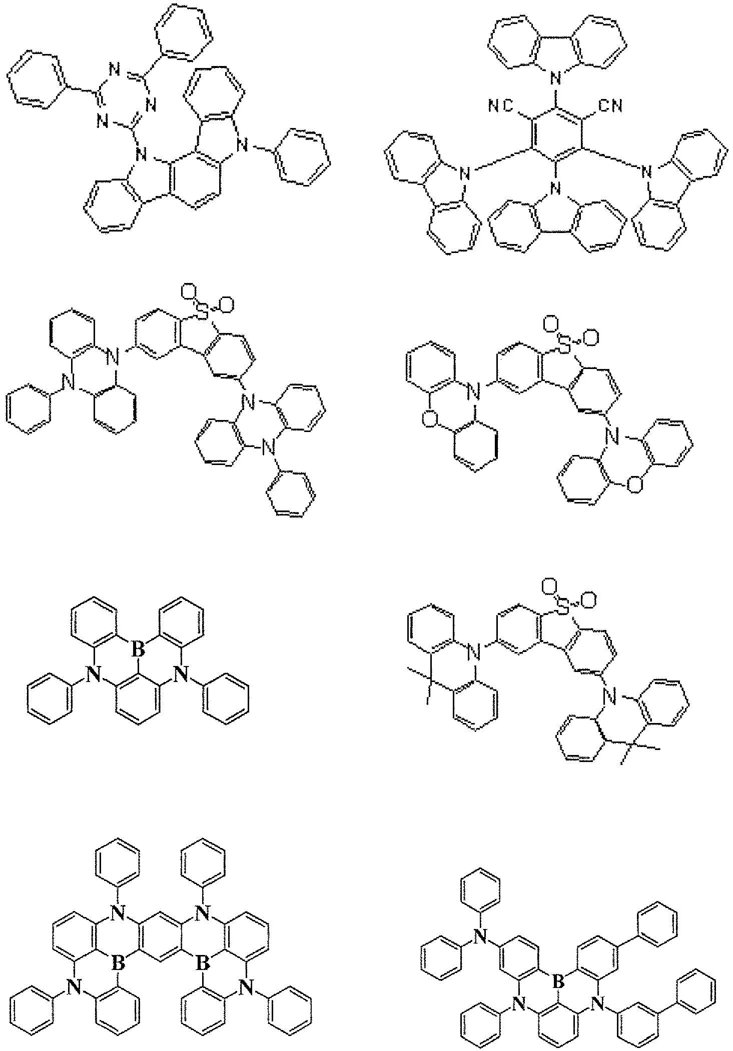

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.1% to 50% and more preferably 1% to 30% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

For the hole blocking layer, a known hole blocking layer material can be used, but it is preferred for the layer to contain the compound represented by the general formula (1).

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq).

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives preferred. Use of arylamine compounds is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitro-substituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

Hereinafter, compounds used in Examples and Comparative Examples are shown below.

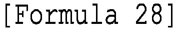

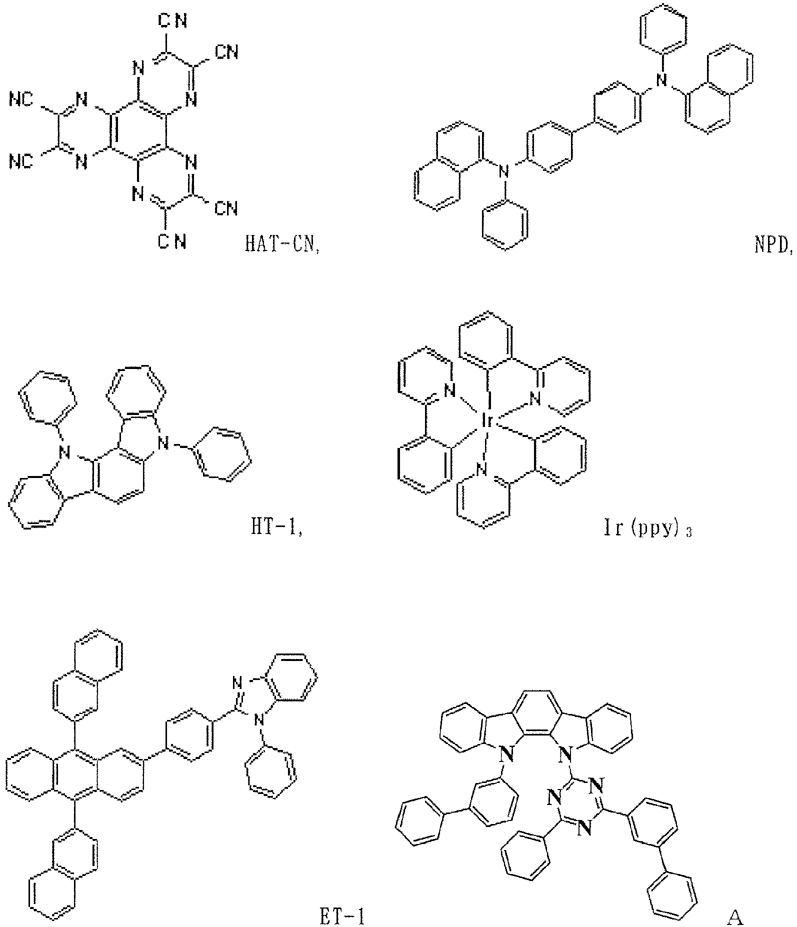

Table 1 shows the 50% weight reduction temperatures (T_{5O}) of compounds (1)-1 and (1)-3, and the like. The 50% weight reduction temperatures were each determined as a temperature at which the weight was reduced by 50% when the temperature was raised to 550°C from room temperature at a rate of 10°C/min in TG-DTA measurement under a nitrogen stream reduced pressure (1 Pa).

**[Table 1]**

| Compound | T₅₀ [°C] |
|---|---|
| (1)-1 | 290 |
| (1)-2 | 310 |
| (1)-3 | 280 |
| (1)-7 | 270 |
| (1)-16 | 296 |
| (2)-2 | 317 |
| (2)-3 | 284 |
| (2)-19 | 267 |
| (2)-21 | 296 |
| Compound A | 271 |

### Example 1

On a glass substrate on which an anode made of ITO with a film thickness of 110 nm was formed, respective thin films were laminated by a vacuum evaporation method at a degree of vacuum of 4.0×10⁻⁵ Pa. First, HAT-CN was formed with a thickness of 25 nm as a hole injection layer on ITO, and next, NPD was formed with a thickness of 30 nm as a hole transport layer. Next, HT-1 was formed with a thickness of 10 nm as an electron blocking layer. Then, compound (1)-1 as a first host, compound (2)-1 as a second host and Ir(ppy)₃ as a light-emitting dopant were co-vapor-deposited from different vapor deposition sources, respectively, to form a light-emitting layer with a thickness of 40 nm. In this case, co-vapor deposition was performed under vapor deposition conditions such that the concentration of Ir(ppy)₃ was 10 wt%, the concentration of a mixed host composed of the first host and the second host was 90 wt% and the weight ratio between the first host and the second host was 30:70. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 2 to 8

Organic EL devices were produced in the same manner as in Example 1 except that compounds shown in Table 1 were used as the first host and the second host instead of those of Example 1.

### Examples 9 to 10

Organic EL devices were produced in the same manner as in Example 1 except that the first host and the second host were mixed in advance to prepare a premixture and the premixture was vapor-deposited from one vapor deposition source in Example 1.

### Example 11

An organic EL device was produced in the same manner as in Example 1 except that co-vapor deposition was performed in Example 1 such that the weight ratio between the first host and the second host was 40:60.

### Example 12

An organic EL device was produced in the same manner as in Example 9 except that the first host and the second host were mixed in advance at a weight ratio of 50:50 to prepare a premixture and the premixture was vapor-deposited from one vapor deposition source in Example 9.

### Comparative Examples 1 to 2

Organic EL devices were produced in the same manner as in Example 1 except that a compound described in Table 1 was used alone as the host in Example 1. The thickness of the light-emitting layer and the concentration of the light-emitting dopant were the same as those in Example 1.

### Comparative Example 3

An organic EL device was produced in the same manner as in Example 1 except that compound A was used as the first host instead of that of Example 1.

### Comparative Example 4

An organic EL device was produced in the same manner as in Example 1 except that compound A was used as the first host and compound (2)-3 was used as the second host instead of those of Examples 9 to 10 where the first host and the second host were mixed in advance to prepare a premixture.

Evaluation results of the produced organic EL devices are shown in Table 1.

In the tables, the luminance, driving voltage, and luminous efficiency are values at a driving current of 20 mA/cm², and they exhibit initial characteristics. LT70 is a time period needed for the initial luminance to be reduced to 70% thereof, and it represents lifetime characteristics.

**[Table 2]**

| | First host | Second host | Mixing ratio (wt%) | Luminance (cd/m²) | Voltage (V) | Power efficiency (lm/W) | LT70 (h) |
|---|---|---|---|---|---|---|---|
| Example 1 | (1)-1 | (2)-1 | 30/70 | 12800 | 3.9 | 51.6 | 1500 |
| Example 2 | (1)-1 | (2)-2 | 30/70 | 12500 | 3.9 | 50.3 | 1450 |
| Example 3 | (1)-1 | (2)-11 | 30/70 | 12900 | 4.0 | 50.7 | 1300 |
| Example 4 | (1)-4 | (2)-3 | 30/70 | 13000 | 3.8 | 53.7 | 1650 |
| Example 5 | (1)-11 | (2)-1 | 30/70 | 12500 | 3.8 | 51.7 | 1300 |
| Example 6 | (1)-16 | (2)-3 | 30/70 | 12600 | 4.0 | 49.5 | 1700 |
| Example 7 | (1)-32 | (2)-3 | 30/70 | 12500 | 3.9 | 50.3 | 1350 |
| Example 8 | (1)-21 | (2)-2 | 30/70 | 13000 | 4.2 | 48.6 | 1600 |
| Example 9 | (1)-3 | (2)-3 | 30/70 | 12500 | 3.9 | 50.3 | 1550 |
| Example 10 | (1)-2 | (2)-2 | 30/70 | 12800 | 3.9 | 51.6 | 1600 |
| Example 11 | (1)-1 | (2)-1 | 40/60 | 13000 | 3.6 | 56.7 | 1300 |
| Example 12 | (1)-3 | (2)-3 | 50/50 | 13100 | 3.4 | 60.5 | 900 |
| Comp. Example 1 | (1)-1 | - | - | 11000 | 3.5 | 49.4 | 350 |
| Comp. Example 2 | (1)-37 | - | - | 9000 | 3.4 | 41.6 | 200 |
| Comp. Example 3 | A | (2)-1 | 30/70 | 10800 | 4.3 | 39.5 | 800 |
| Comp. Example 4 | A | (2)-3 | 30/70 | 11200 | 4.3 | 40.9 | 850 |

## Claims

1. An organic electroluminescent device having a plurality of organic layers between an anode and a cathode, wherein the organic layers comprise at least one light-emitting layer, and the light-emitting layer comprises a compound represented by the following general formula (1), a compound represented by the following general formula (2), and a dopant: 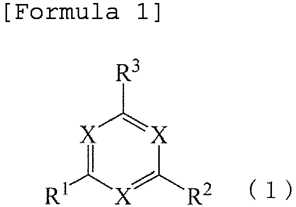 wherein X represents N or CR³, wherein at least one X represents N; R¹ is represented by the following formula (1b) and R² is represented by the following formula (1c); and R³ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, provided that, when the group has a hydrogen atom, the hydrogen atom is optionally replaced with deuterium or halogen; 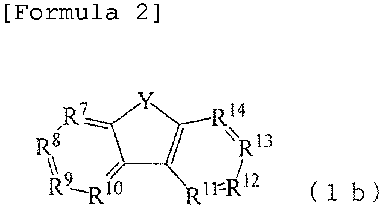 wherein Y represents O, S, NR⁴, or CR⁵R⁶; R⁴ to R⁶ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, provided that, when the group has a hydrogen atom, the hydrogen atom is optionally replaced with deuterium or halogen; and any one of R⁷ to R¹⁴ represents a carbon atom binding to a ring of the general formula (1) and the others each independently represent CR³ or N, and R³ is as described above and R³, when present in a plurality, may be the same or different; 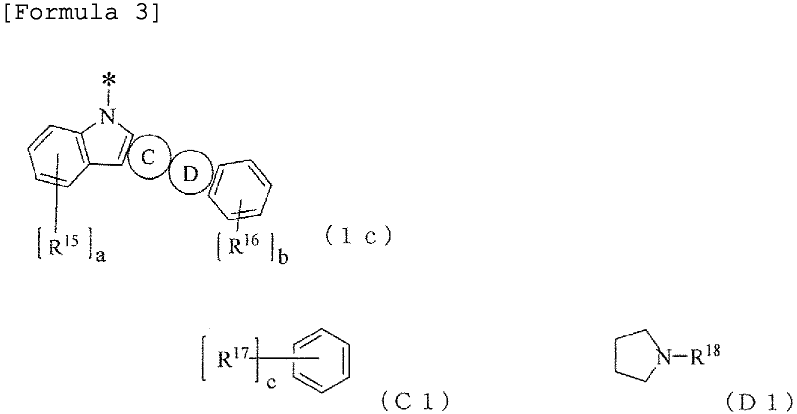 wherein * represents a position for binding to a ring of the general formula (1) and a ring C represents an aromatic ring fused at any positions of two adjacent rings and represented by formula (C1); a ring D represents a 5-membered ring fused at any positions of two adjacent rings and represented by formula (D1); R¹⁵, R¹⁶ and R¹⁷ each independently have the same meaning as R³, and when each present in a plurality, may be each the same or different; a, b and c represent the number of substitutions, a and b each independently represent an integer of 1 to 4, and c represents an integer of 1 to 2; and R¹⁸ has the same meaning as R⁴; 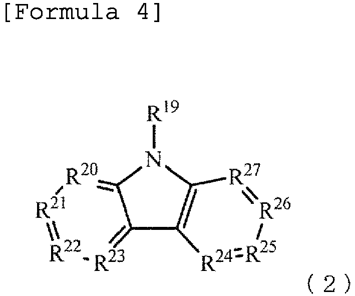 wherein R¹⁹ has the same meaning as R⁴; R²⁰ to R²⁷ each independently represent CR³, CR²⁸ or N, R³ is as described above, wherein at least one thereof is represented by CR²⁸, and R²⁸ is represented by the following formula (2b); and R³ and R²⁸, when each present in a plurality, may be each the same or different; 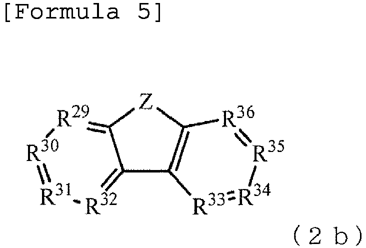 wherein Z represents O, S, NR³⁷, or CR³⁸R³⁹, and R³⁷ to R³⁹ each independently have the same meaning as R⁴; any one of R²⁹ to R³⁶ represents a carbon atom binding to a ring of the general formula (2) and the others each independently represent CR³ or N; and R³ is as described above and R³, when present in a plurality, may be the same or different.

2. The organic electroluminescent device according to claim 1, wherein any of R⁷ or R⁸ in the formula (1b) is linked to a ring of the general formula (1).

3. The organic electroluminescent device according to claim 1, wherein R⁸ in the formula (1b) is linked to a ring of the general formula (1).

4. The organic electroluminescent device according to any one of claims 1 to 3, wherein Y in the formula (1b) represents O or S.

5. The organic electroluminescent device according to any one of claims 1 to 4, wherein the formula (1c) is represented by any of the following formulas (11) to (15) : 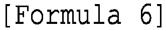 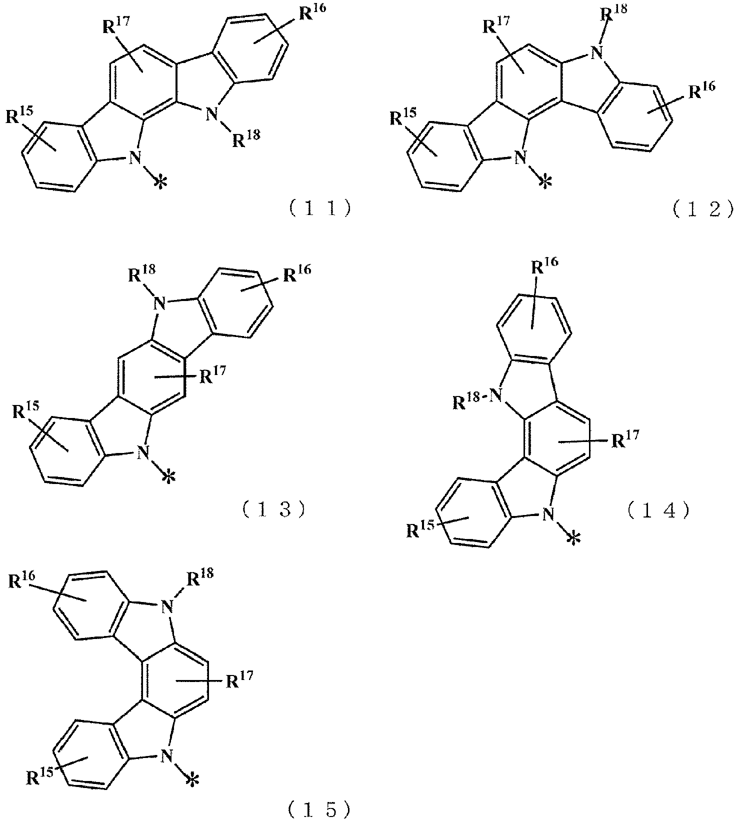 wherein R¹⁵ to R¹⁸ have the same meaning as in the formula (1c).

6. The organic electroluminescent device according to any one of claims 1 to 4, wherein the general formula (2) is represented by any of the general formulas (21) to (29) : 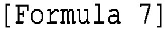 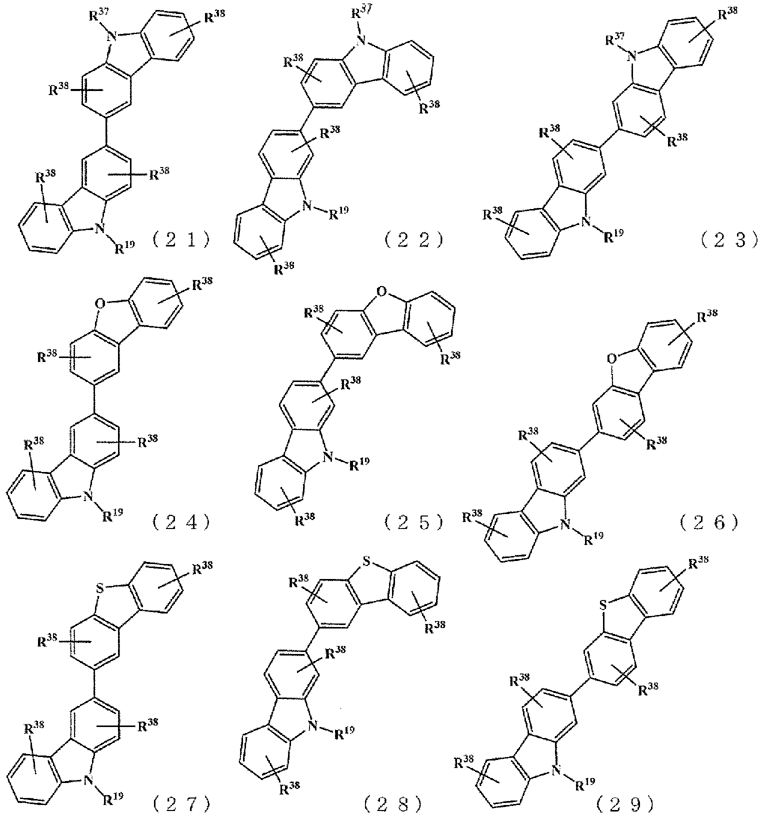 wherein R¹⁹ has the same meaning as in the general formula (2), and R³⁷ and R³⁸ have the same meaning as in the formula (2b).

7. The organic electroluminescent device according to claim 6, wherein the general formula (2) is represented by any of the general formulas (21) to (23).

8. The organic electroluminescent device according to claim 6 or 7, wherein R¹⁹ and R³⁷ in the general formulas (21) to (23) each represent a substituted or unsubstituted aromatic hydrocarbon group having 6 to 10 carbon atoms, or a substituted or unsubstituted linked aromatic group in which two to five of these aromatic rings are linked to each other.

9. The organic electroluminescent device according to any one of claims 1 to 7, wherein the dopant is a phosphorescent dopant or a delayed fluorescence-emitting dopant.

10. A mixed composition comprising a compound represented by the following general formula (1) and a compound represented by the following general formula (2) : 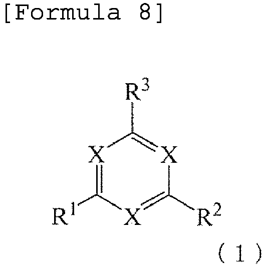 wherein X represents N or CR³, wherein at least one X represents N; R¹ is represented by the following formula (1b) and R² is represented by the following formula (1c); and each R³ independently represents hydrogen, deuterium, halogen, a cyano group, a nitro group, an alkyl group having 1 to 20 carbon atoms, an aralkyl group having 7 to 38 carbon atoms, an alkenyl group having 2 to 20 carbon atoms, an alkynyl group having 2 to 20 carbon atoms, a dialkylamino group having 2 to 40 carbon atoms, a diarylamino group having 12 to 44 carbon atoms, a diaralkylamino group having 14 to 76 carbon atoms, an acyl group having 2 to 20 carbon atoms, an acyloxy group having 2 to 20 carbon atoms, an alkoxy group having 1 to 20 carbon atoms, an alkoxycarbonyl group having 2 to 20 carbon atoms, an alkoxycarbonyloxy group having 2 to 20 carbon atoms, an alkylsulfonyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, provided that, when the group has a hydrogen atom, the hydrogen atom is optionally replaced with deuterium or halogen; 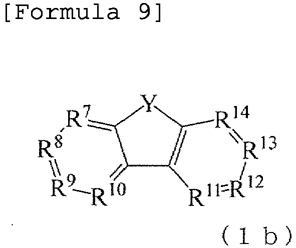 wherein Y represents O, S, NR⁴, or CR⁵R⁶; R⁴ to R⁶ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, provided that, when the group has a hydrogen atom, the hydrogen atom is optionally replaced with deuterium or halogen; and any one of R⁷ to R¹⁴ represents a carbon atom binding to a ring of the general formula (1) and the others each independently represent CR³ or N, and R³ is as described above and R³, when present in a plurality, may be the same or different; 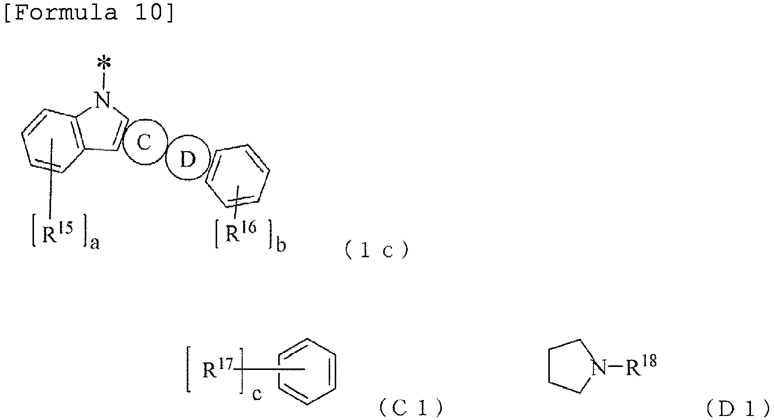 wherein * represents a position for binding to a ring of the general formula (1) and a ring C represents an aromatic ring fused at any positions of two adjacent rings and represented by formula (C1); a ring D represents a 5-membered ring fused at any positions of two adjacent rings and represented by formula (D1); R¹⁵, R¹⁶ and R¹⁷ each independently have the same meaning as R³, and when each present in a plurality, may be each the same or different; a, b and c represent the number of substitutions, a and b each independently represent an integer of 1 to 4, and c represents an integer of 1 to 2; and R¹⁸ has the same meaning as R⁴; 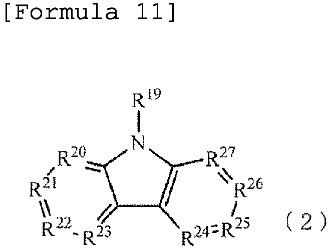 wherein R¹⁹ has the same meaning as R⁴; R²⁰ to R²⁷ each independently represent CR³, CR²⁸ or N, R³ is as described above, wherein at least one thereof is represented by CR²⁸, and R²⁸ is represented by the following formula (2b); and R³ and R²⁸, when each present in a plurality, may be each the same or different; 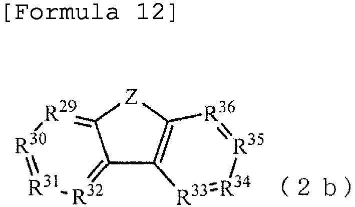 wherein Z represents O, S, NR³⁷, or CR³⁸R³⁹, and R³⁷ to R³⁹ each independently represent hydrogen, deuterium, an alkyl group having 1 to 20 carbon atoms, a substituted or unsubstituted aromatic hydrocarbon group having 6 to 24 carbon atoms, a substituted or unsubstituted aromatic heterocyclic group having 3 to 17 carbon atoms, or a substituted or unsubstituted linked aromatic group in which two to five of these aromatic rings are linked to each other, provided that, when the group has a hydrogen atom, the hydrogen atom is optionally replaced with deuterium or halogen; any one of R²⁹ to R³⁶ represents a carbon atom binding to a ring of the general formula (2) and the others each independently represent CR³ or N; and R³ is as described above and R³, when present in a plurality, may be the same or different.

11. The mixed composition according to claim 10, wherein the general formula (1) represents a compound bound to any of R⁷ or R⁸ in the formula (1b).

12. The mixed composition according to claim 10, wherein the general formula (1) represents a compound bound to R⁸ in the formula (1b).

13. The mixed composition according to any one of claims 10 to 12, wherein a difference in 50% weight reduction temperatures of the compound represented by the general formula (1) and the compound represented by the general formula (2) is within 20°C.

14. A method for producing an organic electroluminescent device, comprising producing a light-emitting layer by use of the mixed composition according to any one of claims 10 to 13.
